(19) Europäisches Patentamt
European Patent Office
Office européen des brevets

(11) EP 3 139 900 B1

(12) # FASCICULE DE BREVET EUROPEEN

(45) Date de publication et mention de la délivrance du brevet:
**26.12.2018 Bulletin 2018/52**

(21) Numéro de dépôt: **15723269.5**

(22) Date de dépôt: **24.04.2015**

(51) Int Cl.:
***A61K 8/37*** *(2006.01)* ***A61Q 1/04*** *(2006.01)*
***A61Q 1/12*** *(2006.01)* ***A61K 8/81*** *(2006.01)*
***A61K 8/85*** *(2006.01)* ***A61K 8/891*** *(2006.01)*
***A61K 8/895*** *(2006.01)* ***A61K 8/02*** *(2006.01)*
***A61K 8/87*** *(2006.01)* ***A61K 8/19*** *(2006.01)*
***A61K 8/34*** *(2006.01)*

(86) Numéro de dépôt international:
**PCT/FR2015/051124**

(87) Numéro de publication internationale:
**WO 2015/170033 (12.11.2015 Gazette 2015/45)**

(54) **PRÉPARATION D'UNE COMPOSITION PULVÉRULENTE / PÂTEUSE COMPRENANT UN GEL D'ÉLASTOMÈRE DE SILICONE, DES PARTICULES SOLIDES ET UNE PHASE LIANTE ET PROCÉDÉ DE TRAITEMENT DES LÈVRES**

HERSTELLUNG EINER PULVERFÖRMIGEN/PASTENZUSAMMENSETZUNG MIT SILIKONELASTOMERGEL, FESTEN PARTIKELN UND EINER BINDEPHASE UND VERFAHREN ZUR LIPPENPFLEGE

PREPARATION OF A PULVERULENT/PASTY COMPOSITION COMPRISING A SILICONE ELASTOMER GEL, SOLID PARTICLES AND A BINDING PHASE AND LIP TREATMENT PROCESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2014 FR 1454104**

(43) Date de publication de la demande:
**15.03.2017 Bulletin 2017/11**

(73) Titulaire: **L'Oréal**
**75008 Paris (FR)**

(72) Inventeurs:
- **DOP, Florence**
  **F-91190 Villiers le Bâcle (FR)**
- **HOTTON, Alexia**
  **F-75011 Paris (FR)**

(74) Mandataire: **Wattremez, Catherine**
**L'Oréal**
**Service DIPI**
**9 Rue Pierre Dreyfus**
**92110 Clichy (FR)**

(56) Documents cités:
**FR-A1- 2 760 635** **FR-A1- 2 922 104**
**FR-A1- 2 945 191** **FR-A1- 2 968 975**
**JP-A- 2005 314 369** **US-A1- 2010 197 805**
**US-A1- 2013 164 235**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte à un procédé de préparation d'une composition se présentant sous forme de poudre, libre ou compactée, ou sous forme de pâte ainsi qu'à un procédé de maquillage et/ou de soin des lèvres la mettant en oeuvre. Plus particulièrement, cette composition comprend au moins un élastomère d'organopolysiloxane véhiculé dans au moins une première huile, des particules solides et une phase liante comprenant au moins une huile non volatile et au moins un composé pâteux.

Les compositions cosmétiques, en particulier de soin et/ou de maquillage, se présentent sous des formes galéniques très diverses, allant des compositions liquides les plus fluides, à des compositions solides, pâteuses voire pulvérulentes.

Dans le domaine des compositions destinées à être appliquées sur les lèvres, elles se présentent habituellement sous la forme de liquides plus ou moins visqueux, comme les brillants à lèvres (ou gloss) notamment, ou encore sous la forme de solides, avec les bâtons de rouge à lèvres ou encore les rouges à lèvres en coupelle.

Une autre caractéristique des compositions à appliquer sur les lèvres est qu'elles permettent d'obtenir des dépôts présentant des degrés de brillance très étendus, puisque certains peuvent être très brillants ou au contraire avoir un aspect satiné, même mat. La présente invention s'intéresse plus particulièrement aux compositions permettant d'obtenir des dépôts mats.

On ne connait pas à l'heure actuelle sur le marché, de compositions destinées à être appliquées sur les lèvres, se présentant sous la forme de poudre. Ce type de galénique est en effet plus approprié et très répandu pour des compositions destinées à des applications sur la peau, comme les fards à paupières ou à joues, les fonds de teint. L'inconvénient majeur de ces compositions pulvérulentes est qu'elles peuvent être relativement inconfortables, du fait de la teneur limitée en composés non volatiles et/ou à la teneur relativement importante en composés volatiles. Si cet inconfort est supportable pour des compositions appliquées sur la peau, il est inacceptable pour des compositions appliquées sur les lèvres.

Afin de résoudre ce problème d'inconfort des compositions pulvérulentes de maquillage de la peau ou des lèvres, la demande internationale WO12/066457, propose des compositions pulvérulentes comprenant au moins 30% en poids d'une phase pulvérulente dans laquelle sont présentes des particules sphériques d'un élastomère de silicone réticulé, et au moins 15% en poids d'une ou plusieurs huiles non volatiles. Le document US2013/0164235 décrit un rouge à lèvres comprenant une importante phase pulvérulente (plus de 30 % en poids), des composés pâteux, des huiles et des élastomères de silicone. Il comprend plus de 5 % en poids de cire.

On constate une amélioration du confort que l'on considère encore insuffisante. En effet, le dépôt sur les lèvres est très sec. En outre, la texture de la composition peut être granuleuse, très cohésive et assez difficile à la prise.

La présente invention a donc pour objectif d'apporter une solution aux problèmes mentionnés ci-dessus. En particulier, elle a pour objet de proposer des compositions sous forme de poudre ou de pâte qui soit facile à prélever et à appliquer. Ces compositions permettent en outre de se déposer sous forme d'un film fin, homogène, mat, confortable et présentant une bonne tenue.

Ainsi, la présente invention a pour objet un procédé de préparation d'une composition anhydre sous forme de poudre ou sous forme de pâte, dans lequel on met en oeuvre les étapes suivantes:

- On prépare 30 à 65 % en poids, par rapport au poids de la composition, de particules solides organiques, minérales ou composites, ainsi que leurs mélanges ;
- On prépare 10 à 40 % en poids par rapport au poids de la composition, d'un mélange comprenant au moins un élastomère d'organopolysiloxane véhiculé dans au moins une première huile non volatile siliconée, ou hydrocarbonée,
- On prépare 10% à 45% en poids d'une phase liante organique comprenant au moins une deuxième huile non volatile, hydrocarbonée ou siliconée, différente ou non de la première huile, au moins un composé pâteux à une teneur comprise entre 5 et 25 % en poids, par rapport au poids de la composition, éventuellement au moins une cire et éventuellement au moins une huile volatile ; la teneur en cire ne dépassant pas 5 % en poids, par rapport au poids de la composition,
- On introduit sous agitation, la phase liante et ledit élastomère d'organopolysiloxane véhiculé, aux particules solides ; l'élastomère d'organopolysiloxane véhiculé étant introduit avec la phase liante ou séparément de celle-ci.

Un autre objet de l'invention consiste en un procédé de maquillage et/ou de soin des lèvres dans lequel on applique la composition selon l'invention.

La composition selon l'invention est homogène, stable (à 4°C, 25°C, 45°C pendant au moins 2 mois) et n'exsude pas d'huile. Elle n'a de plus pas un aspect « cireux » dû notamment à la présence de composé pâteux, qui rendrait le produit plus difficile à la prise. Ainsi, la texture de la composition reste souple, facile à prélever, s'étale également facilement, avec un bon glissant, en un dépôt fin, homogène et couvrant.

La composition obtenue selon l'invention permet d'obtenir par ailleurs un maquillage des lèvres très confortable, non collant, non sec malgré la teneur importante en phase pulvérulente. Il est en outre significativement mat et présente une très bonne tenue de la couleur et de la matité, dans le temps.

De préférence la composition selon l'invention présente l'ensemble de ces avantages combinés.

A noter que dans la description, à moins d'une indication contraire, les bornes indiquées pour un domaine sont incluses dans ce domaine.

Les expressions « au moins un » et « plusieurs » sont utilisées indifféremment.

Par « anhydre », on entend notamment que l'eau n'est de préférence pas ajoutée délibérément dans les compositions mais peut être présente à l'état de trace dans les différents composés utilisés dans les compositions.

Les températures mentionnées plus loin sont indiquées à pression atmosphérique (1,013. $10^5$ Pa).

Par ailleurs, les particules solides, le mélange d'élastomère d'organopolysiloxane véhiculé dans une première huile et la phase liante représentent 100% en poids de la composition.

La composition selon l'invention se présente sous la forme d'une poudre libre ou compactée.

Une poudre compactée désigne plus particulièrement une poudre pressée à l'aide d'une presse manuelle ou mécanique.

Par pâte, on désigne une composition pour laquelle on ne peut pas mesurer de dureté selon la méthode dite du « fil à couper le beurre » à 20°C selon le protocole détaillé ci-dessous car sa dureté est insuffisante et ne permet pas un conditionnement sous forme de bâton. On ne peut également pas mesurer de viscosité selon la méthode qui sera décrite plus bas, car la composition est trop visqueuse.

**Protocole de mesure de la dureté**

La composition sous forme de bâton est conservée à 20°C pendant 24 heures avant la mesure de la dureté.

La mesure est réalisée à 20°C et consiste à couper transversalement un bâton de produit, de préférence cylindrique de révolution, à l'aide d'un fil rigide de tungstène de diamètre 250 μm en déplaçant le fil relativement au stick à une vitesse de 100 mm/min.

La dureté des échantillons de compositions de l'invention, exprimée en $Nm^{-1}$, est mesurée au moyen d'un dynamomètre DFGS2 commercialisé par la société INDELCO-CHATILLON.

La mesure est reproduite trois fois puis moyennée. La moyenne des trois valeurs lues au moyen du dynamomètre mentionné ci-dessus, notée Y, est donnée en grammes. Cette moyenne est convertie en Newton puis divisée par L qui représente la dimension la plus élevée traversée par le fil. Dans le cas d'un bâton cylindrique, L est égal au diamètre (en mètres).

La dureté est convertie en $Nm^{-1}$ par l'équation ci-dessous :

$$(Y \times 10^{-3} \times 9.8)/L$$

**Protocole pour la mesure de la** viscosité :

La mesure de la viscosité est effectuée à 25°C, à l'aide d'un viscosimètre RHEOMAT RM 180 équipé d'un mobile n° 4, la mesure étant effectuée après 10 minutes de rotation du mobile au sein de la composition (temps au terme duquel on observe une stabilisation de la viscosité et de la vitesse de rotation du mobile), à un cisaillement de 200 tours/min (rpm).

Par milieu physiologiquement acceptable, on entend désigner un milieu convenant particulièrement à l'application d'une composition de l'invention sur la peau et les lèvres, ainsi qu'à l'aspect sous lequel la composition est conditionnée.

La présente invention et ses avantages apparaitront plus clairement à la lecture de la description et des exemples qui vont suivre.

**Elastomère d'organopolysiloxane véhiculé dans une première huile non volatile**

Comme indiqué précédemment, la composition selon l'invention comprend au moins un élastomère d'organopolysiloxane (également appelé élastomère siliconé) véhiculé dans au moins une première huile non volatile siliconée ou hydrocarbonée.

Par « véhiculé » on entend au sens de l'invention que l'élastomère est apporté dans la composition sous une forme prédispersée dans au moins une première huile. Plus particulièrement, l'élastomère se trouve sous la forme d'un mélange homogène de particules d'élastomère dispersées dans la première huile, stable pendant au moins 24 heures à 20°C. De préférence, cet élastomère se présente sous la forme d'un gel dans au moins une première huile. En

particulier, une poudre d'élastomère de silicone mise en suspension dans au moins une première huile n'est pas considéré, au sens de l'invention, comme un élastomère d'organopolysiloxane véhiculé dans au moins une première huile.

Par « *élastomère d'organopolysiloxane* » ou « *élastomère siliconé* », on entend un organopolysiloxane souple, déformable, ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement.

Il s'agit plus particulièrement d'un élastomère siliconé réticulé.

Dans ces gels, les particules d'organopolysiloxane peuvent être des particules sphériques ou non-sphériques.

La ou les premières huiles non volatiles siliconées ou hydrocarbonées seront décrites en détails plus loin.

Mais de préférence, l'élastomère d'organopolysiloxane mis en oeuvre dans la composition selon l'invention est véhiculé dans au moins une première huile siliconée non volatile, choisie notamment parmi les huiles siliconées non phénylées, parmi les huiles siliconées phénylées possédant ou non un fragment diméthicone, ou leurs mélanges. Plus avantageusement, la ou les premières huiles non volatiles sont choisies parmi les huiles siliconées non phénylées, en particulier parmi les huiles portant la dénomination INCI « diméthicone ».

L'élastomère présent dans la composition selon l'invention peut être choisi parmi les élastomères non émulsionnants ou émulsionnants.

<u>Elastomère d'organopolysiloxane non émulsionnant</u>

Le terme « *non émulsionnant* » définit des élastomères d'organopolysiloxane ne contenant pas de chaîne hydrophile, et en particulier ne contenant pas de motifs polyoxyalkylène (notamment polyoxyéthylène ou polyoxypropylène), ni de motifs polyglycéryle.

Ainsi, l'élastomère d'organopolysiloxane peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déshydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysiloxane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'élastomère d'organopolysiloxane est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

En particulier, l'élastomère d'organopolysiloxane peut être obtenu par réaction de diméthylpolysiloxane à terminaisons diméthylvinylsiloxy et de méthylhydrogéno-polysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins deux atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25°C allant de 1 à 50000 centistokes, notamment pour être bien miscible avec le composé (B).

Les groupes organiques liés aux atomes de silicium du composé (A) peuvent être des groupes alkyles tels que méthyle, éthyle, propyle, butyle, octyle ; des groupes alkyles substitués tels que 2-phényléthyl, 2-phénylpropyl, 3,3,3-trifluoropropyl ; des groupes aryles tels que phényle, tolyle, xylyle ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (B) est avantageusement un diorganopolysiloxane ayant au moins deux groupes alkényles inférieurs (par exemple en $C_2$-$C_4$) ; le groupe alkényle inférieur peut être choisi parmi les groupes vinyle, allyle, et propényle. Ces groupements alkényles inférieurs peuvent être situés en toute position de la molécule organopolysiloxane mais sont de préférence situés aux extrémités de la molécule organopolysiloxane. L'organopolysiloxane (B) peut avoir une structure à chaîne ramifiée, à chaîne linéaire, cyclique ou en réseau mais la structure en chaîne linéaire est préférée. Le composé (B) peut avoir une viscosité allant de l'état liquide à l'état de gomme. De préférence, le composé (B) a une

viscosité d'au moins 100 centistokes à 25°C.

Outre les groupes alkényles précités, les autres groupes organiques liés aux atomes de silicium dans le composé (B) peuvent être des groupes alkyles tels que méthyle, éthyle, propyle, butyle ou octyle ; des groupes alkyles substitués tels que 2-phényléthyle, 2-phénylpropyle ou 3,3,3-trifluoropropyle ; des groupes aryles tels que phényl, tolyl ou xylyl ; des groupes aryles substitués tels que phényléthyle ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'élastomère d'organopolysiloxane peut être obtenu par réaction de diméthylpolysiloxane à terminaisons diméthylvinylsiloxy et de méthylhydrogénopoly-siloxane à terminaisons triméthylsiloxy, en présence de catalyseur platine.

Selon une autre variante, le composé (B) peut être un composé hydrocarboné insaturé, ayant au moins deux groupes alkényles inférieurs (par exemple en $C_2$-$C_4$) ; le groupe alkényle inférieur peut être choisi parmi les groupes vinyle, allyle, et propényle. Ces groupements alkényles inférieurs peuvent être situés en toute position de la molécule mais sont de préférence situés aux extrémités. A titre d'exemple, on peut citer l'hexadiène, et en particulier le 1,5-hexadiène.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

Comme élastomères non-émulsionnants sphériques, on peut par exemple utiliser ceux vendus sous les dénominations « DC 9040 », « DC 9041 », « DC 9509 », « DC 9505 » par la société Dow Corning.

On peut encore utiliser ceux vendus sous les dénominations « KSG-6 », « KSG-15 », « KSG-16 », « KSG-18 », « KSG-41 », « KSG-42 », « KSG-43 », « KSG-44 », par la société Shin Etsu ; Gransil SR 5CYC gel, Gransil SR DMF 10 gel, Gransil SR DC556 gel de la société Gransil RPS de Grant Industries ; 1229-02-167, 1229-02-168 et « SFE 839 » de la société General Electric.

Selon un mode de réalisation préféré, la composition selon l'invention comprend à titre d'élastomère d'organopolysiloxane véhiculé dans au moins une première huile, un élastomère non-émulsionnant, de préférence sphérique, de préférence choisi parmi les composés vendus sous les dénominations « DC 9040 », « DC 9041 », « DC 9509 », « DC 9505 » par la société Dow Corning.

On peut utiliser selon un mode de réalisation particulier des élastomères en mélange avec une huile siliconée cyclique. On peut citer par exemple le mélange d'organopolysiloxane réticulé/cyclopentasiloxane ou un mélange d'organopolysiloxane réticulé/cyclohexasiloxane comme par exemple le Gransil RPS D5 ou le Gransil RPS D6 de la société Grant Industries.

Elastomère d'organopolysiloxane émulsionnant

Selon un autre mode de réalisation, la composition selon l'invention comprend à titre d'élastomère d'organopolysiloxane véhiculé dans une huile un élastomère émulsionnant.

Par « *élastomère d'organopolysiloxane émulsionnant* », on entend un élastomère d'organopolysiloxane comprenant au moins une chaîne hydrophile, tels que les élastomères d'organopolysiloxane polyoxyalkylénés (polyoxyéthylénés, polyoxypropylénés) et les élastomères de silicone polyglycérolés.

L'élastomère d'organopolysiloxane émulsionnant peut être choisi parmi les élastomères d'organopolysiloxanes polyoxyalkylénés.

L'élastomère d'organopolysiloxane polyoxyalkyléné est un élastomère d'organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'élastomère d'organopolysiloxane polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US 5 236 986 et US 5 412 004.

En particulier, l'organopolysiloxane peut être obtenu par réaction de polyoxyalkylène (notamment polyoxyéthylène et/ou polyoxypropylène) à terminaisons diméthylvinylsiloxy et de méthylhydrogénopolysiloxane à terminaisons trimétylsiloxy, en présence de catalyseur platine.

Les groupes organiques liés aux atomes de silicium du composé (A1) peuvent être des groupes alkyles ayant de 1 à 18 atomes de carbone, tels que méthyle, éthyle, propyle, butyle, octyle, décyle, dodécyle (ou lauryle), myristyle, cétyle, stéaryle ; des groupes alkyles substitués tels que 2-phényléthyle, 2-phénylpropyle, 3,3,3-trifluoropropyle ; des groupes aryles tels que phényléthyle ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères d'organopolysiloxane polyoxyalkylénés peuvent être formés à partir de composés divinyliques, en particulier des polyoxyalkylènes ayant au moins deux groupes vinyliques, réagissant avec des liaisons Si-H d'un polysiloxane.

Des élastomères polyoxyalkylénés sont notamment décrits dans les brevets US 5 236 986, US 5 412 004, US 5 837 793, US 5 811 487 dont le contenu est incorporé par référence.

Comme élastomères d'organopolysiloxane polyoxyalkylénés, on peut utiliser ceux commercialisés sous les dénominations « KSG-21 », « KSG-20 », « KSG-30 », « KSG-31 », « KSG-33 », « KSG-210 », « KSG-310 », « KSG-330 », « KSG-340 » par la société Shin Etsu, « DC9010 », « DC9011 » par la société Dow Corning.

L'élastomère d'organopolysiloxane émulsionnant peut être également choisi parmi les élastomères d'organopolysiloxane polyglycérolés.

L'élastomère d'organopolysiloxane polyglycérolé selon l'invention est un élastomère d'organopolysiloxane pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'élastomère d'organopolysiloxane est obtenu par réaction d'addition réticulation (A2) de diorganopolysiloxane contenant au moins deux hydrogènes liés chacun à un silicium, et (B2) de composés glycérolés ayant au moins deux groupements à insaturation éthylénique, notamment en présence (C2) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A2) est le réactif de base pour la formation d'un élastomère d'organopolysiloxane et la réticulation s'effectue par réaction d'addition du composé (A2) avec le composé (B2) en présence du catalyseur (C2).

Le composé (A2) est en particulier un organopolysiloxane ayant au moins 2 atomes d'hydrogène liés à des atomes de silicium distincts dans chaque molécule.

Le composé (A2) peut présenter toute structure moléculaire, notamment une structure chaîne linéaire ou chaîne ramifiée ou une structure cyclique.

Le composé (A2) peut avoir une viscosité à 25 °C allant de 1 à 50000 centistokes, notamment pour être bien miscible avec le composé (B2).

Les groupes organiques liés aux atomes de silicium du composé (A2) peuvent être des groupes alkyles ayant de 1 à 18 atomes de carbone, tels que méthyle, éthyle, propyle, butyle, octyle, décyle, dodécyle (ou lauryle), myristyle, cétyle, stéaryle ; des groupes alkyles substitués tels que 2-phényléthyle, 2-phénylpropyle, 3,3,3-trifluoropropyle ; des groupes aryles tels que phényle, tolyle, xylyle ; des groupes aryles substitués tels que phényléthyle ; et des groupes hydrocarbonés monovalents substitués tels qu'un groupe époxy, un groupe ester carboxylate, ou un groupe mercapto.

De préférence, ledit groupe organique est choisi parmi les groupes méthyle, phényle et lauryle.

Le composé (A2) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B2) peut être un composé polyglycérolé répondant à la formule (B') suivante :

$C_mH_{2m-1}$-O-[Gly]$_n$-$C_mH_{2m-1}$ (B')

dans laquelle m est un entier allant de 2 à 6, n est un entier allant de 2 à 200, de préférence allant de 2 à 100, de préférence allant de 2 à 50, de préférence allant de 2 à 20, de préférence allant de 2 à 10, et préférentiellement allant de 2 à 5, et en particulier égal à 3 ; Gly désigne ;

$-CH_2-CH(OH)-CH_2-O-$

ou

$-CH_2-CH(CH_2OH)-O-$

Avantageusement, la somme du nombre de groupements éthyléniques par molécule du composé (B2) et du nombre d'atomes d'hydrogène liés à des atomes de silicium par molécule du composé (A2) est d'au moins 4.
Il est avantageux que le composé (A2) soit ajouté en une quantité telle que le rapport moléculaire entre la quantité totale d'atomes d'hydrogène liés à des atomes de silicium dans le composé (A2) et la quantité totale de tous les groupements à insaturation éthylénique dans le composé (B2) soit compris dans la gamme de 1/1 à 20/1.
Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.
Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).
L'élastomère d'organopolysiloxane polyglycérolé est véhiculé sous forme de gel dans au moins une huile hydrocarbonée et/ou une huile siliconée. Dans ces gels, l'élastomère polyglycérolé est souvent sous forme de particules non sphériques.
Comme élastomères d'organopolysiloxane polyglycérolés, on peut utiliser ceux vendus sous les dénominations « KSG-710 », « KSG-810 », « KSG-820 », « KSG-830 », « KSG-840 » par la société Shin Etsu.
De manière préférée, l'élastomère de silicone véhiculé dans au moins une première huile est non émulsionnant, c'est-à-dire dénué de chaîne hydrophile, et en particulier dénué de motifs polyoxyalkylénés et de motifs polyglycéryle.
Avantageusement, l'élastomère d'organopolysiloxane considéré selon l'invention est choisi parmi les élastomères d'organopolysiloxane non émulsionnants sphériques.
Plus particulièrement, l'élastomère d'organopolysiloxane est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence de catalyseur platine (C).
De façon avantageuse, la composition selon l'invention comprend une teneur en mélange élastomère(s) d'organopolysiloxane véhiculé(s) dans au moins une première huile non volatile, variant de de 15 à 35 % en poids, par rapport au poids de la composition.
Selon une variante préférée de la présente invention, la composition comprend une teneur en élastomère d'organopolysiloxane dans la composition, exprimée en élastomère d'organopolysiloxane, varie de 2 à 10 % en poids de la composition.

## Particules solides

La composition selon l'invention comprend par ailleurs, des particules solides, organiques, minérales ou composites, ainsi que leurs mélanges.
Plus particulièrement, ces particules sont choisies parmi les particules solides colorées ou parmi les charges, ainsi que leurs mélanges

## Particules solides colorées

Plus particulièrement, les particules solides colorées sont des pigments minéraux, organiques ou composites, ainsi que leurs mélanges.
Par « *pigments* », il faut comprendre des particules blanches ou colorées, minérales et/ou organiques, insolubles dans une solution aqueuse, destinées à colorer et/ou opacifier la composition et/ou le dépôt réalisé à partir de la composition.
Les pigments peuvent être choisis parmi les pigments, en particulier minéraux, monochromes, les laques organiques, les nacres, les pigments à effet optiques, comme les pigments réfléchissants et les pigments goniochromatiques.

Les pigments minéraux peuvent être choisis parmi les pigments d'oxyde métallique, les oxydes de chrome, les oxydes de fer, le dioxyde de titane, les oxydes de zinc, les oxydes de cérium, les oxydes de zirconium, le violet de manganèse, le bleu de prusse, le bleu outremer, le bleu ferrique, le bleu outremer, l'hydrate de chrome et leurs mélanges.

Il peut également s'agir de pigments ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

Les laques organiques sont des pigments organiques formés d'un colorant fixé sur un substrat.

Elles peuvent être par exemple choisies parmi :

- le carmin de cochenille ;
- les pigments organiques de colorants azoïques, anthraquinoniques, indigoïdes, xanthéniques, pyréniques, quinoliniques, de triphénylméthane, de fluorane. Parmi les pigments organiques, on peut notamment citer ceux connus sous les dénominations suivantes: D&C Blue n° 4, D&C Brown n° 1, D&C Green n° 5, D&C Green n° 6, D&C Orange n° 4, D&C Orange n° 5, D&C Orange n°10, D&C Orange n° 11, D&C Red n° 6, D&C Red n° 7, D&C Red n° 17, D&C Red n°21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.
- les sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Les laques organiques peuvent également être supportées par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

On peut également citer les colorants liposolubles tels que par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les matériaux chimiques correspondant à chacune des matières colorantes organiques citées précédemment sont mentionnés dans l'ouvrage « International Cosmetic Ingrédient Dictionnary and Handbook », Edition 1997, pages 371 à 386 et 524 à 528, publié par « The Cosmetic, Toiletry, and Fragrance Association », dont le contenu est incorporé dans la présente demande par référence.

Les pigments peuvent également avoir subi un traitement hydrophobe.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Par « nacre », au sens de la présente demande, on entend des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites en tant que pigment interférentiel dans la première composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut également contenir à titre de particules colorées, au moins un matériau à effet optique spécifique.

Cet effet est différent d'un simple effet de teinte conventionnel, c'est-à-dire unifié et stabilisé tel que produit par les particules colorées classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet.

De manière avantageuse, la composition selon l'invention présente une teneur en particule(s) colorée(s) variant de 5 à 25 % en poids, par rapport au poids de la composition.

**Charges**

Les charges sont plus particulièrement organiques, minérales ou mixtes, et peuvent être présentes seules ou en mélange.

Par « charges », il faut comprendre les particules incolores ou blanches, solides de toutes formes, qui se présentent sous une forme insoluble et dispersée dans le milieu de la composition, quelle que soit la température à laquelle la composition est fabriquée. De nature minérale ou organique, elles permettent de modifier la rhéologie ou la texture de la composition, de lui conférer du corps ou de la rigidité.

Les charges peuvent être de toute forme, par exemple plaquettaire, sphérique, oblongue, fibreuse, ou tout autre forme intermédiaire entre ces dernières, quelle que soit la forme cristallographique (par exemple feuillet, cubique, hexagonale, orthorhombique, etc).

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Comme exemples de charges minérales, on peut citer le talc ; le mica ; la silice, pyrogénée ou non, éventuellement traitée hydrophile ou hydrophobe ; la perlite ; le kaolin ; la bentone ; les microsphères de silice creuses ; le carbonate de calcium précipité ; le carbonate et l'hydrocarbonate de magnésium ; l'hydroxyapatite ; le nitrure de bore ; les microcapsules de verre ou de céramique ; les composites de silice et de dioxyde de titane, comme la série TSG commercialisée par Nippon Sheet Glass.

Parmi les charges de type silice pyrogénée, éventuellement traitée hydrophile ou hydrophobe, et de préférence traitée hydrophobe, on peut mentionner par exemple les charges de type « Silica diméthyl silylate » (nom INCI selon le CTFA).

Les groupements hydrophobes peuvent notamment être des groupements diméthylsilyloxyl ou polydiméthylsiloxane, qui sont par exemple obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL 972®", "AEROSIL R974®" par la société Degussa, "CAB-O-SIL TS-610®", "CAB-O-SIL TS-720®" par la société Cabot.

Comme exemples de charges organiques, on peut citer les poudres de polyamide (Nylon® Orgasol de chez Atochem), de polyéthylène, de polyméthacrylate de méthyle, les poudres de polytétrafluoroéthylène (Téflon), de copolymères d'acide acrylique (Polytrap de la société Dow Corning), la lauroyl lysine, les microsphères creuses polymériques telles que celles de chlorure de polyvinylidène/acrylonitrile comme l'Expancel (Nobel Industrie), la poudre de copolymère hexamethylene diisocyanate/Trimethylol hexyllactone (Plastic Powder de Toshiki), les microbilles de résine de silicone (Tospearl de Toshiba par exemple) les cires micronisées synthétiques ou naturelles, les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore® L 200 (Chemdal Corporation), les poudres de polyuréthane, en particulier, les poudres de polyuréthane réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple, sous la dénomination de PLASTIC POWDER D-400® ou PLASTIC POWDER D-800® de la société TOSHIKI, et leurs mélanges.

On peut également citer en tant que charge organique, les poudres d'organopolysiloxane différent de l'élastomère de polyorganosiloxane véhiculé dans au moins une première huile décrit précédemment. Plus particulièrement, on peut citer les poudres d'élastomère d'organopolysiloxane réticulé enrobé de résine de silicone, notamment de résine silsesquioxane, comme décrit par exemple dans le brevet US 5538793,

De telles poudres d'élastomère sont vendues sous les dénominations KSP-100®, KSP-101®, KSP-102®, KSP-103®, KSP-104® et KSP-105® par la société SHIN ETSU ; on peut également citer des poudres d'élastomère d'organopolysiloxane réticulé enrobées de résine de silicone telles que des poudres de silicone hybride fonctionnalisée par des groupes fluoroalkyle, notamment vendues sous la dénomination "KSP-200" par la société Shin Etsu ; ou des poudres de silicones hybrides fonctionnalisées par des groupes phényl, notamment vendues sous la dénomination "KSP-300" par la société Shin Etsu.

Selon un mode de réalisation particulier de l'invention, la composition comprend une teneur en charge(s) organique(s), minérale(s) ou composite(s), ou leurs mélanges, représente de 10 à 40 % en poids, par rapport au poids de la composition.

## Phase liante

Comme indiqué précédemment, la composition selon l'invention comprend une phase liante renfermant au moins une deuxième huile non volatile, hydrocarbonée ou siliconée, différente ou non de la première huile. De préférence, la ou les deuxièmes huiles sont différentes de la ou des premières huiles.

A noter que la ou les première(s) et deuxième(s) huiles, hydrocarbonées ou siliconées, sont liquides à 25°C et pression atmosphérique.

Il est également précisé que les huiles hydrocarbonées ou siliconées sont des composés non miscibles à l'eau. Par « non miscible », on entend que le mélange de la même quantité d'eau et d'huile ne conduit pas à une solution monophasique homogène, à 25°C et pression atmosphérique.

Par « non volatile » on désigne des composés dont le point éclair est supérieur ou égal à 49°C. Le point d'éclair est mesuré en coupelle fermée à à l'aide d'un appareil dit de Pensky-Martens (*Closed Cup*).

La description des huiles hydrocarbonées ou siliconées non volatiles qui vont suivre, conviennent autant pour les première(s) huile(s) et deuxième(s) huile(s).

**Huiles non volatiles hydrocarbonées**

A titre d'huiles non volatiles hydrocarbonées convenable au sens de l'invention, on peut citer les huiles hydrocarbonées apolaires ou polaires, ainsi que leurs mélanges.

Par « huile hydrocarbonée», on entend une huile formée essentiellement, voire constituée, d'atomes de carbone et d'hydrogène, et éventuellement d'atomes d'oxygène, d'azote, et ne contenant pas d'atome de silicium ou de fluor.

Huiles hydrocarbonées non volatiles apolaires

Ces huiles peuvent être d'origine végétale, minérale ou synthétique.

Par « huile apolaire » au sens de la présente invention, on entend une huile dont le paramètre de solubilité à 25°C, $\delta_a$, est égal à 0 $(J/cm^3)^{½}$.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ainsi que les forces d'interactions de KEESOM entre dipôles induits et dipôles permanents;
- $\delta_h$ caractérise les forces d'interactions spécifiques (type liaisons hydrogène, acide/base, donneur/accepteur, etc.) ; et
- $\delta_a$ est déterminé par l'équation : $\delta_a = (\delta_p^2 + \delta_h^2)^{½}$.

Les paramètres $\delta_p$, $\delta_h$, $\delta_D$ et $\delta_a$ sont exprimés en $(J/cm^3)^{½}$.

De façon préférée, l'huile hydrocarbonée apolaire non volatile peut être choisie parmi les hydrocarbures linéaires ou ramifiés, d'origine minérale ou synthétique tels que :

- l'huile de paraffine ou ses dérivés,
- le squalane,
- l'isohexadécane
- l'isoeicosane,
- l'huile de naphtalène,
- les polybutènes tels que par exemple L'INDOPOL H-100 (de masse molaire ou MW=965 g/mol), L'INDOPOL H-300 (MW=1340 g/mol), L'INDOPOL H-1500 (MW=2160g/mol) commercialisés ou fabriqués par la société AMOCO,
- les polyisobutènes, les polyisobutènes hydrogénés tels que par exemple le Parléam® commercialisé par la société NIPPON OIL FATS, le PANALANE H-300 E commercialisé ou fabriqué par la société AMOCO (MW =1340 g/mol), le VISEAL 20000 commercialisé ou fabriqué par la société SYNTEAL (MW=6000 g/mol), le REWOPAL PIB 1000 commercialisé ou fabriqué par la société WITCO (MW=1000 g/mol), ou encore le PARLEAM LITE commercialisé par NOF Corporation,
- les copolymères décène/butène, les copolymères polybutène/polyisobutène notamment l'Indopol L-14,
- les polydécènes et les polydécènes hydrogénés tels que par exemple : le PURESYN 10 (MW=723 g/mol), le PURESYN 150 (MW=9200 g/mol) commercialisés ou fabriqués par la société MOBIL CHEMICALS, ou encore le PURESYN 6 commercialisé par EXXONMOBIL CHEMICAL),
- et leurs mélanges.

De préférence, la ou les huiles hydrocarbonées apolaires non volatiles, si la composition en contient, sont choisies parmi les polybutènes, hydrogénés ou non, les polyisobutènes hydrogénés ou non, les polydécènes hydrogénés ou non, ainsi que leurs mélanges.

De préférence, la composition selon l'invention comprend au moins une huile hydrocarbonée non volatile apolaire, en particulier celles mentionnées ci-dessus.

Huiles non volatiles hydrocarbonées polaires

Ces huiles sont donc formées essentiellement, voire constituées, d'atomes de carbone et d'hydrogène, et

comprennent éventuellement un ou plusieurs atomes d'oxygène, d'azote, mais ne contiennent pas d'atome de silicium ou de fluor.

Elles peuvent donc contenir des fonctions alcool, ester, éther, acide carboxylique, amine et/ou amide.

De préférence, les huiles non volatiles hydrocarbonées polaires sont exemptes, outre de silicium, de fluor ; d'hétéroatomes tels que N et P. Les huiles hydrocarbonées sont par conséquent distinctes des huiles siliconées et fluorées.

Dans le cas présent, la ou les huiles non volatiles hydrocarbonées polaires comprennent au moins un atome d'oxygène.

En particulier, la ou les huiles non volatiles hydrocarbonées polaires comprennent au moins une fonction alcool (il s'agit alors d'une « huile alcool ») ou au moins une fonction ester (il s'agit alors d'une « huile ester »). A noter que les huiles esters peuvent notamment être hydroxylées.

La composition peut comprendre une ou plusieurs huiles non volatiles hydrocarbonées, en particulier choisies parmi :

- les alcools en $C_{10}$-$C_{26}$, plus particulièrement en $C_{10}$-$C_{24}$, et de préférence en $C_{12}$-$C_{22}$, saturés ou non, ramifiés ou non, plus particulièrement les monoalcools.

Plus particulièrement, les alcools en $C_{10}$-$C_{26}$ sont des monoalcools gras, de préférence ramifiés lorsqu'ils comprennent au moins 16 atomes de carbone.

A titre d'exemples d'alcools gras pouvant être utilisés selon l'invention, on peut citer les alcools gras linéaires ou ramifiés, d'origine synthétique, ou encore naturelle comme par exemple les alcools provenant de matières végétales (coprah, palmiste, palme...) ou animales (suif...).

Bien entendu, d'autres alcools à longue chaîne peuvent également être utilisés, comme par exemple les éther-alcools ou bien encore les alcools dits de Guerbet.

Enfin, on peut également utiliser certaines coupes plus ou moins longues d'alcools d'origine naturelle, comme par exemple coco ($C_{12}$ à $C_{16}$) ou suif ($C_{16}$ à $C_{18}$) ou des composés type diols ou cholesterol.

A titre d'exemples particuliers d'alcools gras utilisables à titre préféré, on peut notamment citer l'alcool laurique, isostéarylique, oléique, le 2-butyloctanol, le 2-undécyl pentadécanol, l'alcool 2-hexyldécylique, l'alcool isocétylique, l'octyldodécanol et leurs mélanges.

Selon un mode de réalisation avantageux de l'invention, l'alcool est choisi parmi l'octyldodécanol.

- les monoesters, les diesters, les triesters, optionnellement hydroxylés, d'un acide mono ou polycarboxylique en $C_2$-$C_8$ et d'un alcool en $C_2$-$C_8$.

En particulier :

* les monoesters d'un acide carboxylique en $C_2$-$C_8$ et d'un alcool en $C_2$-$C_8$, optionnellement hydroxylés,
* les diester d'un diacide carboxylique en $C_2$-$C_8$ et d'un alcool en $C_2$-$C_8$, optionnellement hydroxylés ; tels que le diisopropyl adipate, le diéthyl-2 hexyl adipate, le dibutyl adipate, ou le succinate de 2-diéthyl-hexyle,
* les triesters d'un triacide carboxylique en $C_2$-$C_8$ et d'un alcool en $C_2$-$C_8$, optionnellement hydroxylés, tels que les esters d'acide citrique, tels que le trioctyle citrate, triéthylcitrate, l'acétyltributyl citrate, le tributyl citrate, l'acétyltributyl citrate,

- les esters d'un polyol en $C_2$-$C_8$ et d'un ou plusieurs acides carboxyliques en $C_2$-$C_8$, tels que les diesters de glycol et de monoacides, tels que le diheptanoate de néopentylglycol, le dioctanoate de propylène glycol, ou les triesters de glycérol et de monoacides tel que la triacétine.

- les huiles ester, en particulier ayant au moins 18 atomes de carbone et encore plus particulièrement entre 18 et 70 atomes de carbone.

A titre d'exemples, on peut citer les mono-, di- ou tri- esters.

Les huiles esters peuvent être hydroxylées ou non.

Ainsi, l'huile ester non volatile peut être choisie par exemple parmi :

* les monoesters comprenant au moins 18 atomes de carbone et encore plus particulièrement ayant entre 18 et 40 atomes de carbone au total, en particulier les monoesters, de formule $R_1COOR_2$ dans laquelle $R_1$ représente le reste d'un acide gras linéaire ou ramifié ou aromatique comportant de 4 à 40 atomes de carbone, saturé ou non, et $R_2$ représente une chaîne hydrocarbonée notamment ramifiée contenant de 4 à 40 atomes de carbone à condition

que la somme des atomes de carbone des radicaux $R_1$ et $R_2$ soit supérieure ou égale à 18, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, le néopentanoate d'octyledodécyle, le stéarate d'octyl-2 dodécyle, l'érucate d'octyl-2 dodécyle, l'isostéarate d'isostéaryle, les benzoate d'alkyle en $C_{12}$-$C_{15}$, comme le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle.

De préférence, il s'agit des esters de formule $R_1COOR_2$ dans laquelle $R_1$ représente le reste d'un acide gras linéaire ou ramifié comportant de 4 à 40 atomes de carbone et $R_2$ représente une chaîne hydrocarbonée notamment ramifiée contenant de 4 à 40 atomes de carbone, $R_1$ et $R_2$ étant tels que la somme des atomes de carbone des radicaux $R_1$ et $R_2$ soit supérieure ou égale à 18.

Encore plus particulièrement, l'ester comprend entre 18 et 40 atomes de carbone au total.

* les monoesters, en particulier ayant au moins 18 atomes de carbone et encore plus particulièrement de 18 à 22 atomes de carbone, d'acide gras comme notamment d'acide lanolique, d'acide oléique, d'acide laurique, d'acide stéarique, et de diols tels que les glycols, comme le monoisostéarate de propylène glycol.
* les diesters, notamment ayant au moins 18 atomes de carbone et encore plus particulièrement comprenant entre 18 et 60 atomes de carbone au total, en particulier entre 18 et 50 atomes de carbone au total. On peut notamment utiliser les diesters de diacide carboxylique et de monoalcools comprenant plus de 8 atomes de carbone, tels que, de préférence, le diisostéaryle malate, le diisostéaryle adipate ; ou les diesters de glycol et de monoacides carboxyliques, tels que le diheptanoate de néopentylglycol, le diisononanoate de diéthylèneglycol ; ou le polyglycéryle-2 diisostéarate (notamment tel que le composé vendu sous la référence commerciale DERMOL DGDIS par la société Akzo).
* les monoesters et les diesters hydroxylés, de préférence ayant un nombre total de carbone d'au moins 18 atomes de carbone et encore plus particulièrement allant de 18 à 70, comme le polyglycéryl-3 diisostéarate, le lactate d'isostéaryle, l'octylhydroxystéarate, l'hydroxystéarate d'octyldodécyle, le malate de diisostéaryle, le stéarate de glycérine.
* les triesters, notamment ayant au moins 35 atomes de carbone et encore plus particulièrement comprenant entre 35 et 70 atomes de carbone au total, en particulier tel que les triesters de triacide carboxylique, tels que le triisostéaryle citrate, ou le tridécyl trimellitate, ou les triesters de glycérol et de monoacides carboxyliques tel que le triisostéarate de polyglycérol-2.
* les tétraesters, notamment ayant au moins 35 atomes de carbone et encore plus particulièrement ayant un nombre total de carbone allant de 35 à 70, tel que les tétraesters de penthaérythritol ou de polyglycérol et d'un monoacide carboxylique, par exemple tels que le tétrapélargonate de pentaérythrityle, le pentaérythrityle de tetraisostéarate, le tétraisononanoate de pentaérythrityle, le tri décyl-2 tétradécanoate de glycéryle, le tétraisostéarate de polyglycéryle-2 ou encore le tétra décyl-2 tétradécanoate de pentaérythrityle.
* les polyesters obtenus par condensation de dimère et/ou trimère d'acide gras insaturé et de diols tels que ceux décrits dans la demande de brevet FR 0 853 634. En particulier, le dimère d'acide gras insaturé peut comprendre de 28 à 44 atomes de carbone, 2 fonctions acides carboxylique et 2 à 4 insaturations ; le trimère d'acide gras insaturé peut comprendre de 42 à 66 atomes de carbone, 3 fonctions acide carboxylique, ainsi que 3 à 6 insaturations. De préférence, on utilise un dimère d'acide gras insaturé, en particulier ayant 36 atomes de carbone et 2 fonctions acide carboxylique. Des mélanges de dimères et de trimères d'acide gras insaturé et/ou d'acide gras insaturé (non polymérisé donc correspondant à un monomère) peuvent également être mis en oeuvre. Par ailleurs, le diol comprend de 2 à 10 atomes de carbone, et deux fonctions hydroxyle. En particulier, on peut citer les esters de l'acide dilinoléique et du 1,4- butanediol ou du propanediol. On peut notamment mentionner à ce titre le polymère commercialisé par Biosynthis sous la dénomination Viscoplast 14436H (nom INCI : dilinoleic acid/butanediol copolymer), ou encore les copolymères de polyols et de dimères diacides, et leurs esters, tels que le Hailucent ISDA.
* les esters et polyesters de dimère diol et d'acide mono- ou dicarboxylique, tels que les esters de dimère diol et d'acide gras et les esters de dimère diols et de dimère diacide carboxylique, en particulier pouvant être obtenus à partir d'un dimère diacide carboxylique dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$, tels que les esters de diacides dilinoléiques et de dimères diols dilinoléiques, par exemple tels que ceux commercialisés par la société NIPPON FINE CHEMICAL sous la dénomination commerciale LUSPLAN DD-DA5® et DD-DA7®.
* les polyesters résultant de l'estérification d'au moins un triglycéride d'acide(s) carboxylique(s) hydroxylé(s) par un acide monocarboxylique aliphatique et par un acide dicarboxylique aliphatique, éventuellement insaturé comme l'huile de ricin d'acide succinique et d'acide isostéarique commercialisée sous la référence Zénigloss par Zénitech.
* les huiles hydrocarbonées végétales telles que les triglycérides d'acides gras (liquides à température ambiante),

notamment d'acides gras ayant au moins 7 atomes de carbone et encore plus particulièrement ayant de 7 à 40 atomes de carbone, tels que les triglycérides des acides heptanoïque ou octanoïque ou l'huile de jojoba, en particulier, on peut citer les triglycérides saturés tels que le triglycéride d'acide carptique/caprylique et leurs mélanges, par exemple tel que celui commercialisé sous la référence Myritol 318 de Cognis, le triheptanoate de glycéryle, le trioctanoate de glycérine, les triglycérides d'acide en $C_{18-36}$ tels que ceux commercialisés sous la référence DUB TGI 24 commercialisé par Stéarineries Dubois), et les triglycérides insaturés tels que l'huile de ricin, l'huile d'olive, l'huile de ximénia, l'huile de pracaxi.

- les copolymères vinylpyrrolidone/1-héxadécène, comme par exemple celui vendu sous la dénomination ANTARON V-216 (également appelé Ganex V216) par la société ISP (MW=7300 g/mol).

- les acides gras en $C_{12}$-$C_{26}$, de préférence en $C_{12}$-$C_{22}$, de préférence insaturés, tels que l'acide oléique, l'acide linoléique, l'acide linolénique, et leurs mélanges.

- les carbonates de di-alkyle, les 2 chaînes alkyles pouvant être identiques ou différentes, tels que le dicaprylyl carbonate commercialisé sous la dénomination Cetiol CC®, par Cognis.

- et leurs mélanges.

De préférence, la ou les huiles hydrocarbonées polaires non volatiles, si la composition en contient, sont choisies parmi les huiles esters, et en particulier les monoesters, les diesters, hydroxylés ou non, comprenant au moins 18 atomes de carbone au total, ainsi que leurs mélanges.

De préférence, la composition selon l'invention comprend au moins une huile non volatile polaire, de manière avantageuse choisie parmi celles mentionnées ci-dessus.

**Huiles non volatiles siliconées**

Selon une variante de l'invention, la phase liante comprend au moins un composé siliconé non volatile liquide à 25°C et pressions atmosphérique choisi parmi les huiles siliconées non phénylées, parmi les huiles siliconées phénylées possédant ou non un fragment diméthicone, ou leurs mélanges, identiques ou différentes de la ou des premières huiles non volatiles siliconées

Huiles siliconées non volatiles non phénylées

L'expression «huile siliconée non phénylée» ou «huile siliconée non phényle» désigne une huile siliconée n'ayant pas de substituant phényle.

Des exemples représentatifs de ces huiles siliconées non phénylées non volatiles qui peuvent être mentionnés, comprennent des polydiméthylsiloxanes ; des alkyldiméthicones ; des vinylméthylméthicones ; et également des silicones modifiées avec des groupes aliphatiques et/ou avec des groupes fonctionnels tels que des groupes hydroxyle, thiol et/ou amine.

A noter que «diméthicone» (nom INCI) correspond à un polydiméthylsiloxane (nom chimique).

En particulier, ces huiles peuvent être choisies parmi les huiles siliconées non volatiles non phénylées suivantes :

- des polydiméthylsiloxanes (PDMS),
- des PDMS comprenant des groupes aliphatiques, en particulier alkyle, ou alcoxy, qui sont pendants et/ou à la fin de la chaîne silicone ; ces groupes comprenant chacun de 2 à 24 atomes de carbone. A titre d'exemple on peut citer la cétyldiméthicone commercialisée sous la référence commerciale ABIL WAX 9801 d'Evonik Goldschmidt,

- des PDMS comprenant au moins un groupe aliphatique et /ou au moins un groupe fonctionnel tel que les groupes hydroxyle, thiol et/ou amine,
- des polysiloxanes modifiés avec des acides gras, des alcools gras ou des polyoxyalkylènes, et des mélanges de ceux-ci.
- les silicones cycliques comme l'octaméthyl cyclotétrasiloxane, le dodécaméthyl cyclohexasiloxane, le cyclopentasiloxane.

L'huile siliconée non phénylée non volatile est de préférence choisie parmi des huiles diméthicones non volatiles.

De préférence, ces huiles siliconées non phénylées non volatiles sont choisies parmi des

polydiméthylsiloxanes ; des alkyldiméthicones et également des PDMS comprenant au moins un groupe aliphatique, en particulier alkyle en $C_2$-$C_{24}$, et/ou au moins un groupe fonctionnel tel que les groupes hydroxyle, thiol et/ou amine.

L'huile siliconée non phénylée peut être choisie en particulier parmi des silicones de formule (I') :

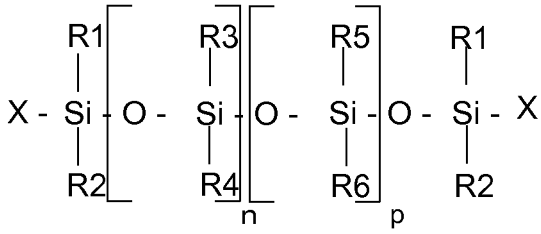

(I')

dans laquelle :

$R_1$, $R_2$, $R_5$ et $R_6$ sont, ensemble ou séparément, un radical alkyle contenant 1 à 6 atomes de carbone,
$R_3$ et $R_4$ sont, ensemble ou séparément, un radical alkyle contenant 1 à 6 atomes de carbone, un radical vinyle, un radical amine ou un radical hydroxyle,
X est un radical alkyle contenant de 1 à 6 atomes de carbone, un radical hydroxyle ou un radical amine,
n et p sont des entiers choisis de manière à avoir un composé fluide, en particulier dont la viscosité à 25°C est comprise entre 2 centistokes (cSt) et 800 000 cSt.

En tant qu'huiles siliconées non phénylées non volatiles qui peuvent être utilisées selon l'invention, il peut être mentionné celles pour lesquelles :

- les substituants $R_1$ à $R_6$ et X représentent un groupe méthyle, et p et n sont tels que la viscosité soit de 500 000 cSt (soit 450000 mPa.s), par exemple le produit commercialisé sous le nom SE30 par l'entreprise General Electric, le produit commercialisé sous le nom AK 500000 par l'entreprise Wacker, le produit commercialisé sous le nom Mirasil DM 500 000 par l'entreprise Bluestar, et le produit commercialisé sous le nom Dow Corning 200 Fluid 500 000 cSt (soit 450000 mPa.s), par l'entreprise Dow Corning,
- les substituants $R_1$ à $R_6$ et X représentent un groupe méthyle, et p et n sont tels que la viscosité soit de 60 000 cSt (54000 mPa.s), par exemple le produit commercialisé sous le nom Dow Corning 200 Fluid 60000 CS par l'entreprise Dow Corning, et le produit commercialisé sous le nom Wacker Belsil DM 60 000 par l'entreprise Wacker.
- les substituants $R_1$ à $R_6$ et X représentent un groupe méthyle, et p et n sont tels que la viscosité soit de 100 cSt (soit 90 mPa.s), soit de 350 cSt (soit 315 mPa.s) , par exemple les produits commercialisés respectivement sous les noms Belsil DM100, Dow Corning 200 Fluid 350 CS, par l'entreprise Dow Corning.
- les substituants $R_1$ à $R_6$ représentent un groupe méthyle, le groupe X représente un groupe hydroxyle, et n et p sont tels que la viscosité soit de 700 cSt (630 mPa.s), par exemple le produit commercialisé sous le nom Baysilone Fluid T0.7 par l'entreprise Momentive.
- les substituants $R_1$ à $R_6$ et X représentent un groupe méthyle, et p et n sont tels que la viscosité soit de 5 cSt, par exemple le produit commercialisé sous la dénomination XIAMETER® PMX-200 SILICONE FLUID 5 CS de Dow Corning.

Conviennent aussi la dodécaméthylpentasiloxane, la décaméthyltétrasiloxane.

Huiles siliconées phénylées non volatiles

L'expression « huile siliconée phénylée » ou « huile phénylsilicon(é)e » désigne une huile siliconée ayant au moins un substituant phényle.

Ces huiles silicones phénylées peuvent être choisies parmi celles possédant en outre au moins un fragment diméthicone, ou parmi celles n'en possédant pas.

Selon l'invention, un fragment diméthicone correspond au motif suivant : $-Si(CH_3)_2-O-$.

L'huile siliconée phénylée non volatile peut ainsi être choisie parmi :

a) les huiles phénylsiliconées possédant ou non un fragment diméthicone correspondant à la formule suivante (I) :

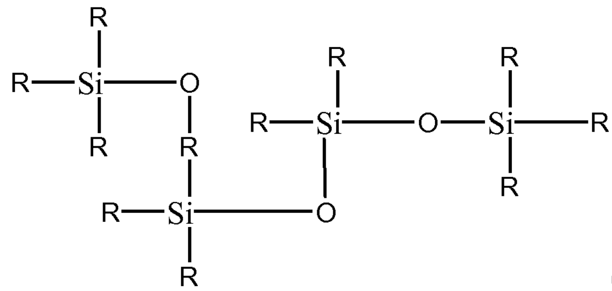

(I)

dans laquelle les groupes R, monovalent ou divalent, représentent, indépendamment les uns des autres, un méthyle ou un phényle, à condition qu'au moins un groupe R représente un phényle.

De préférence, dans cette formule, l'huile phénylsiliconée comprend au moins trois groupes phényle, par exemple au moins quatre, au moins cinq ou au moins six.

b) les huiles phénylsiliconées possédant ou non un fragment diméthicone correspondant à la formule suivante (2) :

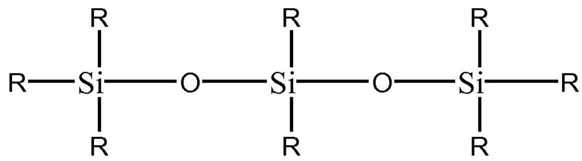

(II)

dans laquelle les groupes R représentent, indépendamment les uns des autres, un méthyle ou un phényle, à condition qu'au moins un groupe R représente un phényle.

De préférence, dans cette formule, le composé de formule (II) comprend au moins trois groupes phényle, par exemple au moins quatre ou au moins cinq.

Des mélanges de composés différents phénylorganopolysiloxanes précédemment décrits peuvent être utilisés.

Des exemples qui peuvent être mentionnés comprennent des mélanges de triphényl-, tétraphényl- ou pentaphényl-organopolysiloxanes.

Parmi les composés de formule (II), on peut citer plus particulièrement les huiles phénylsiliconées ne possédant pas de fragment diméthicone correspondant à la formule (II) dans laquelle au moins 4 ou au moins 5 radicaux R représentent un radical phényle les radicaux restant représentant des méthyle.

De telles huiles phénylsiliconées non volatiles sont de préférence le triméthylpentaphényl-trisiloxane, ou le tétraméthyl-tétraphényl-trisiloxane. Elles sont en particulier commercialisées par Dow Corning sous la référence PH-1555 HRI ou Dow Corning 555 Cosmetic Fluid (nom chimique : 1,3,5-triméthyl-1,1,3,5,5-pentaphényltrisiloxane; nom INCI : triméthyl-pentaphényltrisiloxane), ou le tétraméthyl-tétraphényl-trisiloxane commercialisé sous la référence Dow Corning 554 Cosmetic Fluid par Dow Corning peut également être utilisé.

Elles correspondent notamment aux formules suivantes (III), (III') :

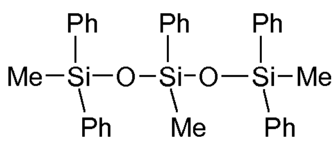

(III)

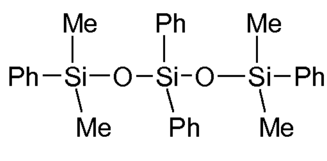

(III')

dans lesquelles Me représente méthyle, Ph représente phényle.

c) les huiles phénylsiliconées possédant au moins un fragment diméthicone correspondant à la formule suivante (IV) :

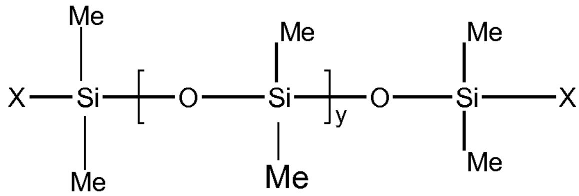

(IV)

dans laquelle Me représente méthyle, y est entre 1 et 1 000 et X représente $-CH_2-CH(CH_3)(Ph)$.

d) les huiles phénylsiliconées correspondant à la formule (V) ci-dessous, et des mélanges de celles-ci :

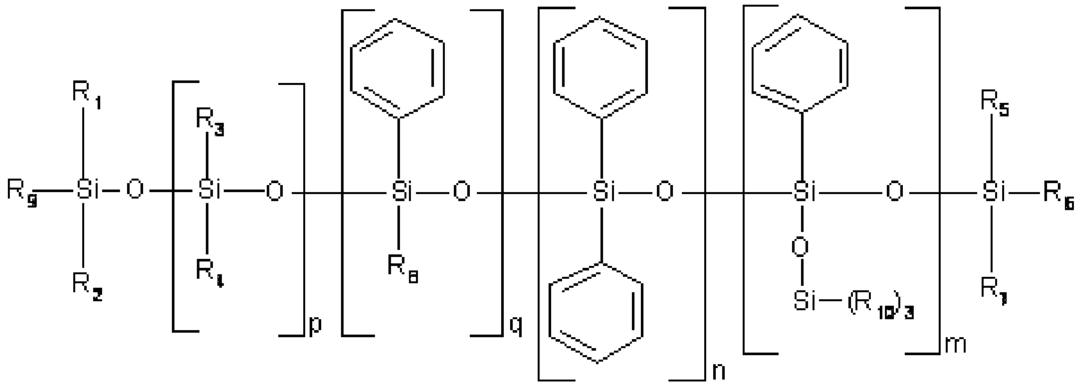

(V)

dans laquelle :

- $R_1$ à $R_{10}$, indépendamment les uns des autres, sont des radicaux hydrocarbonés en $C_1$-$C_{30}$, saturés ou insaturés, linéaires, cycliques ou ramifiés, de préférence saturés ou insaturés, linéaires ou ramifiés,
- m, n, p et q sont, indépendamment les uns des autres, des entiers compris entre 0 et 900, à condition que la somme m+n+q soit différente de 0.

De préférence, la somme m+n+q est comprise entre 1 et 100. De préférence, la somme m+n+p+q est comprise entre 1 et 900 et de préférence entre 1 et 800. De préférence, q est égal à 0.

De préférence, $R_1$ à $R_{10}$, indépendamment les uns des autres, représentent un radical alkyle en $C_1$-$C_{30}$, linéaire ou ramifié, de préférence en $C_1$-$C_{20}$, plus particulièrement en $C_1$-$C_{16}$, ou un radical aryle en $C_6$-$C_{14}$ et en particulier en $C_{10}$-$C_{13}$, monocyclique ou polycyclique, ou un radical aralkyle de préférence dont la partie alkyle est en $C_1$-$C_3$.

De préférence, $R_1$ à $R_{10}$ peuvent chacun représenter un radical méthyle, éthyle, propyle, butyle, isopropyle, décyle, dodécyle ou octadécyle, ou en variante un radical phényle, tolyle, benzyle ou phénéthyle. $R_1$ à $R_{10}$ peuvent en particulier être identiques, et en outre peuvent être un radical méthyle.

Selon un premier mode de réalisation plus particulier de la formule (V) on peut citer :

**i)** les huiles phénylsiliconées possédant ou non au moins un fragment diméthicone correspondant à la formule (VI) ci-dessous, et des mélanges de celles-ci :

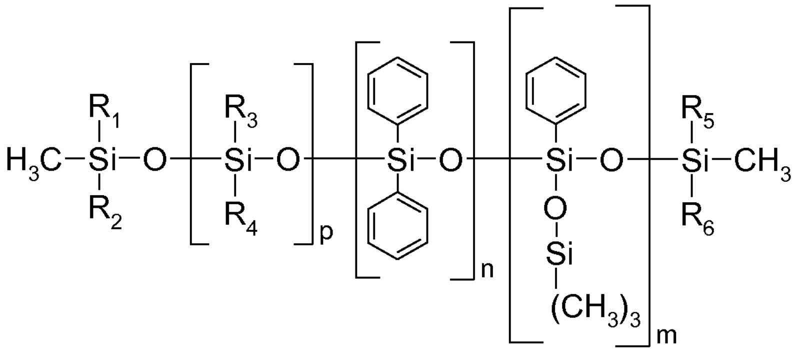

(VI)

dans laquelle :

- $R_1$ à $R_6$, indépendamment les uns des autres, sont des radicaux hydrocarbonés en $C_1$-$C_{30}$ saturés ou insaturés, linéaires, cycliques ou ramifiés, de préférence saturés ou insaturés, linéaires ou ramifiés, un radical aryle, de préférence en $C_6$-$C_{14}$, ou un radical aralkyle dont la partie alkyle est en $C_1$-$C_3$.
- m, n et p sont, indépendamment les uns des autres, des entiers compris entre 0 et 100, à condition que la somme n+m soit comprise entre 1 et 100.

De préférence, $R_1$ à $R_6$, indépendamment l'un de l'autre, représentent un radical alkyle, en $C_1$-$C_{30}$, de préférence en $C_1$-$C_{20}$, en particulier en $C_1$-$C_{16}$, ou un radical aryle en $C_6$-$C_{14}$ monocyclique (de préférence en $C_6$) ou polycyclique et en particulier en $C_{10}$-$C_{13}$, ou un radical aralkyle (de préférence la partie aryle est en $C_6$ ; la partie alkyle est en $C_1$-$C_3$).
De préférence, $R_1$ à $R_6$ peuvent chacun représenter un radical méthyle, éthyle, propyle, butyle, isopropyle, décyle, dodécyle ou octadécyle, ou en variante un radical phényle, tolyle, benzyle ou phénéthyle.
$R_1$ à $R_6$ peuvent en particulier être identiques, et en outre peuvent être un radical méthyle. De préférence, m = 1 ou 2 ou 3, et/ou n = 0 et/ou p = 0 ou 1 peut être appliqué, dans la formule (VI).
Selon un mode de réalisation particulier, l'huile siliconée phénylée non volatile est choisie parmi les huiles siliconées phénylées possédant au moins un fragment diméthicone.
De préférence, de telles huiles correspondent à des composés de formule (VI) dans laquelle :

**A)** m=0 et n et p sont indépendamment l'un de l'autre, des entiers compris entre 1 et 100.

De préférence $R_1$ à $R_6$ sont des radicaux méthyle.
Selon ce mode de réalisation, l'huile siliconée est de préférence choisie parmi une diphényldiméthicone telle que KF-54 de Shin Etsu, KF54HV de Shin Etsu, KF-50-300CS de Shin Etsu, KF-53 de Shin Etsu, KF-50-100CS de Shin Etsu.

B) p est compris entre 1 et 100, la somme n+m est comprise entre 1 et 100, et n=0.

Ces huiles phénylsiliconées possédant ou non au moins un fragment diméthicone correspondant plus particulièrement à la formule (VII) ci-dessous :

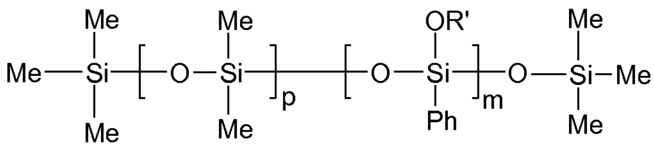

(VII)

dans laquelle Me est méthyle et Ph est phényle, OR' représente un groupe $-OSiMe_3$ et p vaut 0 ou est compris entre 1 et 1000, et m est compris entre 1 et 1000. En particulier, m et p sont tels que le composé (VII) soit une huile non volatile.
Selon un premier mode de réalisation de silicone phénylée non volatile possédant au moins un fragment diméthicone, p est compris entre 1 et 1000. m est plus particulièrement tel que la composé (VII) soit une huile non volatile. Il peut être utilisé, par exemple, la triméthylsiloxyphényldiméthicone, commercialisée en particulier sous la référence Belsil PDM 1000 par l'entreprise Wacker.

Selon un deuxième mode de réalisation de silicone phénylée non volatile ne possédant pas de fragment diméthicone, p est égal à 0. m est compris entre 1 et 1000, et en particulier, est tel que le composé (VII) soit une huile non volatile

Il peut être utilisé, par exemple, le phényltriméthylsiloxytrisiloxane, commercialisé en particulier sous la référence Dow Corning 556 Cosmetic Grade Fluid (DC556).

**ii)** les huiles phénylsiliconées non volatiles ne possédant pas de fragment diméthicone correspondant à la formule (VIII) ci-dessous, et des mélanges de ceux-ci :

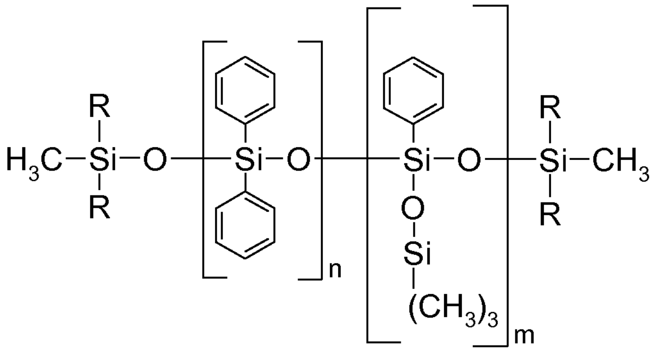

(VIII)

dans laquelle :

- R, indépendamment les uns des autres, représentent un radical hydrocarboné en $C_1$-$C_{30}$ saturé ou insaturé, linéaire, cyclique ou ramifié, de préférence saturé ou insaturé, linéaire ou ramifié ; plus particulièrement R représentent un radical alkyle en $C_1$-$C_{30}$, un radical aryle, de préférence en $C_6$-$C_{14}$, ou un radical aralkyle dont la partie alkyle est en $C_1$-$C_3$.
- m et n sont, indépendamment l'un de l'autre, des entiers compris entre 0 et 100, à condition que la somme n+m soit comprise entre 1 et 100.

De préférence, R, indépendamment les uns des autres, représentent un radical alkyle en $C_1$-$C_{30}$, linéaire ou ramifié, et en particulier en $C_1$-$C_{20}$, en particulier en $C_1$-$C_{16}$ un radical aryle en $C_6$-$C_{14}$ monocyclique ou polycyclique et en particulier en $C_{10}$-$C_{13}$, ou un radical aralkyle de préférence la partie aryle est en $C_6$ et la partie alkyle est en $C_1$-$C_3$.

De préférence, les R peuvent chacun représenter un radical méthyle, éthyle, propyle, butyle, isopropyle, décyle, dodécyle ou octadécyle, ou en variante un radical phényle, tolyle, benzyle ou phénéthyle.

Les R peuvent en particulier être identiques, et en outre peuvent être un radical méthyle.

De préférence, m = 1 ou 2 ou 3, et/ou n = 0 et/ou p = 0 ou 1 peut être appliqué, dans la formule (VIII).

Selon un mode de réalisation préféré, n est un entier compris entre 0 et 100 et m est un entier compris entre 1 et 100, à condition que la somme n+m soit comprise entre 1 et 100, dans la formule (VIII). De préférence R est un radical méthyle.

Selon un mode de réalisation, une huile phénylsilicone de formule (VIII) ayant une viscosité à 25 °C comprise entre 5 et 1500 $mm^2/s$ (c'est-à-dire, de 5 à 1500 cSt), et de préférence ayant une viscosité comprise entre 5 et 1000 $mm^2/s$ (c'est-à-dire 5 à 1000 cSt) peut être utilisée.

Selon ce mode de réalisation, l'huile phénylsiliconée non volatile est de préférence choisie parmi des phényltriméthicones (lorsque n=0) telles que DC556 de Dow Corning (22,5 cSt), ou encore parmi l'huile diphénylsiloxyphényltriméthicone (lorsque m et n sont compris entre 1 et 100) telle que KF56 A de Shin Etsu, l'huile Mirasil PTM silicone de Bluestar Silicone (28 cSt). Les valeurs entre parenthèses représentent les viscosités à 25°C.

(e) les huiles phénylsiliconées possédant ou non au moins un fragment diméthicone correspondant à la formule suivante, et des mélanges de celles-ci :

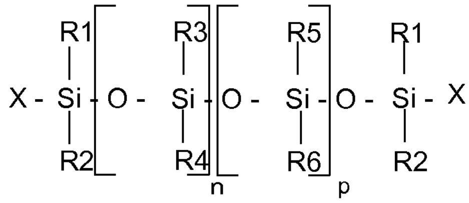

(IX)

dans laquelle :

$R_1$, $R_2$, $R_5$ et $R_6$ sont, identiques ou non, un radical alkyle contenant 1 à 6 atomes de carbone,
$R_3$ et $R_4$ sont, identiques ou non, un radical alkyle contenant de 1 à 6 atomes de carbone ou un radical aryle (de préférence en $C_6$-$C_{14}$), à condition qu'au moins l'un de $R_3$ et $R_4$ soit un radical phényle,
X est un radical alkyle contenant de 1 à 6 atomes de carbone, un radical hydroxyle ou un radical vinyle,
n et p étant un entier supérieur ou égal à 1, choisi de manière à conférer à l'huile une masse moléculaire moyenne en poids inférieure à 200 000 g/mole, de préférence inférieure à 150 000 g/mole et plus préférablement inférieure à 100 000 g/mole.

f) et un mélange de celles-ci.

Si la composition comprend au moins une deuxième huile différente de la première, non volatile siliconée, celle-ci est de préférence choisie parmi les huiles non volatiles siliconées phénylées possédant ou non un fragment diméthicone, par exemple les huiles correspondant à celles décrites sous le paragraphe d), plus particulièrement celles de formule (VI) sous i), de préférence les silicones décrites sous B) de formule (VII).

Selon un mode de réalisation particulièrement avantageux de l'invention, la phase liante comprend au moins une deuxième huile différente de la première huile.

Conformément à un mode de réalisation particulièrement préféré de l'invention, la phase liante comprend en tant que deuxième(s) huile(s), au moins une huile non volatile hydrocarbonée polaire ou apolaire, ou siliconée, ainsi que leurs mélanges.

Parmi les huiles hydrocarbonées non volatiles apolaires, conviennent particulièrement les polybutènes, hydrogénés ou non, les polyisobutènes hydrogénés ou non, les polydécènes hydrogénés ou non, ainsi que leurs mélanges, et de préférence parmi les polyisobutènes hydrogénés ou non, et leurs mélanges.

Parmi les huiles hydrocarbonées non volatiles polaires conviennent particulièrement les huiles esters, et en particulier les monoesters, les diesters, hydroxylés ou non, comprenant au moins 18 atomes de carbone au total.

Parmi les huiles non volatiles siliconées, on préfère utiliser les huiles non volatiles siliconées phénylées possédant ou non un fragment diméthicone, par exemple les huiles décrites sous le paragraphe d), plus particulièrement celles de formule (VI) sous i), de préférence les silicones décrites sous B) de formule (VII).

De préférence, la phase liante comprend en tant que deuxièmes huiles, au moins une huile hydrocarbonée non volatile, et au moins une huile siliconée non volatile.

Avantageusement, la teneur en deuxième(s) huile(s) hydrocarbonée(s) non volatile(s), siliconée(s) non volatile(s), ou leurs mélanges, varie de 5 à 40 % en poids, de préférence de 10 à 20 % en poids par rapport au poids de la composition.

## Composé pâteux

La phase liante de la composition selon l'invention comprend également au moins un composé pâteux à 25°C et pressions atmosphérique.

A noter que ce composé pâteux est non miscible à l'eau.

Par « *pâteux* » au sens de la présente invention, on entend un composé à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97 % en poids du composé pâteux. Cette fraction liquide à 23°C représente de préférence entre 15 et 85 %, de préférence encore entre 40 et 85 % en poids.

Au sens de l'invention, la température de fusion correspond à la température du pic le plus endothermique observé en analyse thermique (DSC) telle que décrite dans la norme ISO 11357-3 ; 1999. Le point de fusion d'un composé pâteux peut être mesuré à l'aide d'un calorimètre à balayage différentiel (DSC), par exemple le calorimètre

vendu sous la dénomination « MDSC 2920 » par la société TA Instruments.

Le protocole de mesure est le suivant :

Un échantillon de 5 mg de composé pâteux disposé dans un creuset est soumis à une première montée en température allant de -20°C à 100°C, à la vitesse de chauffe de 10°C/minute, puis est refroidi de 100°C à -20°C à une vitesse de refroidissement de 10°C/minute et enfin soumis à une deuxième montée en température allant de -20°C à 100°C à une vitesse de chauffe de 5°C/minute. Pendant la deuxième montée en température, on mesure la variation de la différence de puissance absorbée par le creuset vide et par le creuset contenant l'échantillon de corps gras pâteux en fonction de la température. Le point de fusion du composé pâteux est la valeur de la température correspondant au sommet du pic de la courbe représentant la variation de la différence de puissance absorbée en fonction de la température.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par ce dernier pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (DSC), tel que le calorimètre vendu sous la dénomination MDSC 2920 par la société TA instrument, avec une montée en température de 5 ou 10°C par minute, selon la norme ISO 11357-3 ; 1999.

L'enthalpie de fusion du composé pâteux est la quantité d'énergie nécessaire pour faire passer le composé pâteux de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 2°C constitué d'une fraction liquide et d'une fraction solide.

La fraction liquide du composé pâteux mesurée à 32°C représente de préférence de 30 à 100 % en poids du composé pâteux, de préférence de 50 à 100 %, de préférence encore de 60 à 100 % en poids du composé pâteux. Lorsque la fraction liquide du composé pâteux mesurée à 32°C est égale à 100 %, la température de la fin de la plage de fusion du composé pâteux est inférieure ou égale à 32°C.

La fraction liquide du corps composé mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux peut en particulier être choisi parmi les composés pâteux synthétiques et les corps gras d'origine végétale.

Le ou les composés pâteux peuvent être en particulier choisis parmi :

- la lanoline et ses dérivés, tels que l'alcool de lanoline, les lanolines oxyéthylénées, la lanoline acétylée, les esters de lanoline tels que le lanolate d'isopropyle, les lanolines oxypropylénées ;
- la vaseline (également appelée petrolatum),
- les éthers de polyol choisis parmi les éthers de pentaérythritol et de polyalkylène glycol en $C_2$-$C_4$, les éthers d'alcool gras et de sucre, et leurs mélanges. Par exemple, on peut citer l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société VEVY, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja,
- les composés siliconés polymères ou non,
- les composés fluorés polymères ou non,
- les polymères vinyliques, notamment :
    - les homopolymères et les copolymères d'oléfines,
    - les homopolymères et copolymères de diènes hydrogénés,
    - les oligomères linéaires ou ramifiés, homo ou copolymères de (méth)acrylates d'alkyles ayant de préférence un groupement alkyle en $C_8$-$C_{30}$,
    - les oligomères homo et copolymères d'esters vinyliques ayant des groupements alkyles en $C_8$-$C_{30}$, et
    - les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$.

Parmi les polyéthers liposolubles, on considère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne disposés en blocs ayant un poids moléculaire moyen de 1000 à 10000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par AKZO NOBEL.

- les esters et polyesters,

Parmi les esters, on considère notamment :

- les esters d'un oligomère de glycérol, notamment les esters de diglycérol, en particulier les condensats d'acide adipique et de diglycérol, pour lesquels une partie des groupes hydroxyles des glycérols ont réagi avec un mélange d'acides gras tels que l'acide stéarique, l'acide caprique, l'acide stéarique, l'acide isostéarique et l'acide 12-hydroxystéarique, comme par exemple le bis-diglyceryl polyacyladipate-2 commercialisé sous la référence SOFTISAN® 649 par la société Sasol,
- les homopolymères de vinyl ester ayant des groupements alkyles en $C_8$-$C_{30}$, tels que le polyvinyl laurate (notamment vendu sous la référence Mexomère PP par la société Chimex),
- le propionate d'arachidyle commercialisé sous la marque Waxenol 801 par ALZO,
- les esters de phytostérol,
- les triglycérides d'acides gras et leurs dérivés,
- les esters de pentaérythritol,
- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate; de tels esters peuvent être notamment choisis parmi les esters de nomenclature INCI suivante : le bis-béhényl/isostéaryl/phytostéryl dimerdilinoléyle dimerdilinoléate (Plandool G), le phytostéryl/isostéryl/cétyl/stéaryl/béhényl dimerdilinoléate (Plandool H ou Plandool S) et leurs mélanges,
- les beurres d'origine végétale comme le beurre de mangue, tel que celui commercialisé sous la référence Lipex 203 par la société AARHUSKARLSHAMN, le beurre de karité, en particulier celui dont le nom INCI est Butyrospermum Parkii Butter, tel que celui commercialisé sous la référence Sheasoft® par la société AARHUSKARLSHAMN, le beurre de cupuacu (Rain forest RF3410 de la société Beraca Sabara), le beurre de murumuru (RAIN FOREST RF3710 de la société Beraca Sabara), le beurre de cacao ; ainsi que la cire d'orange comme, par exemple, celle qui est commercalisée sous la référence Orange Peel Wax par la société Koster Keunen,
- les huiles végétales totalement ou partiellement hydrogénées, comme par exemple l'huile de soja hydrogénée, l'huile de coprah hydrogénée, l'huile de colza hydrogénée, les mélanges d'huiles végétales hydrogénées tels que le mélange d'huile végétale hydrogénée de soja, coprah, palme et colza, par exemple le mélange commercialisé sous la référence Akogel® par la société AARHUSKARLSHAMN (nom INCI Hydrogenated Vegetable Oil), l'huile de jojoba partiellement hydrogénée isomérisée trans fabriquée ou commercialisée par la société Desert Whale sous la référence commerciale Iso-Jojoba-50®, l'huile d'olive partiellement hydrogénée comme, par exemple, le composé commercialisé sous la référence Beurrolive par la société Soliance,
- les esters d'huile de ricin hydrogénée, comme l'huile de ricin hydrogénée dimère dilinoléate par exemple le RISOCAST-DA-L vendu par KOKYU ALCOHOL KOGYO, l'isostéarate d'huile de ricin hydrogénée par exemple le SALACOS HCIS (V-L) vendu par NISSHIN OIL,
- et leurs mélanges.

De préférence, les composés pâteux convenables à la mise en oeuvre de l'invention, sont choisis parmi les composés hydrocarbonés et comprennent, outre les atomes de carbone et d'hydrogène, au moins des atomes d'oxygène. Les composés pâteux ne comprennent donc pas d'atome de silicium ni d'atome de fluor.

Selon un mode de réalisation préféré, la phase liante comprend au moins un composé pâteux, avantageusement choisi parmi la lanoline et ses dérivés, les esters, ou leurs mélanges. En particulier, le ou les composés pâteux sont choisis parmi la lanoline et ses dérivés, les esters d'oligomères de glycérol, les beurres d'origine végétale, les huiles végétales totalement ou partiellement hydrogénées, les esters d'huiles de ricin hydrogéné, ou leurs mélanges.

Comme indiqué précédemment, la teneur en composé pâteux à 25°C et pression atmosphérique représente de 5 à 25 % en poids, de préférence de 5 à 15 % en poids, par rapport à la composition.

**Cires**

La composition selon l'invention peut éventuellement comprendre au moins une cire.

Par « *cire* » au sens de la présente invention, on entend un composé lipophile, solide à température ambiante (25°C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (DSC), par exemple le calorimètre vendu sous la dénomination DSC 30 par la société METLER.

De façon préférée, le protocole de mesure est le suivant :

Un échantillon de 5 mg de cire disposé dans un creuset est soumis à une première montée en température allant de -20°C à 100°C, à la vitesse de chauffe de 10°C/minute, puis est refroidi de 100°C à -20°C à une vitesse de refroidissement de 10°C/minute et enfin soumis à une deuxième montée en température allant de -20°C à 100°C à une vitesse de chauffe de 5°C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

La cire peut notamment présenter une dureté allant de 0,05 MPa à 15 MPa, et de préférence allant de 6 MPa à 15 MPa. La dureté est déterminée par la mesure de la force en compression mesurée à 20°C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées ou fluorées et être d'origine végétale, minérale, animale et/ou synthétique.

En particulier, les cires présentent une température de fusion supérieure à 30°C et mieux supérieure à 45°C.

Cire apolaire

Par « *cire apolaire* », au sens de la présente invention, on entend une cire dont le paramètre de solubilité $\delta_a$ à 25°C tel que défini ci-après est égal à 0 $(J/cm^3)^{½}$.

Les cires apolaires sont en particulier les cires hydrocarbonées constituées uniquement d'atomes de carbone et d'hydrogène et exemptes d'hétéroatomes tel que N, O, Si et P.

A titre illustratif des cires apolaires convenant à l'invention, on peut notamment citer les cires hydrocarbonées comme les cires microcristallines, les cires de paraffines, l'ozokérite, les cires de polyéthylène, les microcires notamment de polyéthylène.

Cire polaire

Par « *cire polaire* », au sens de la présente invention, on entend une cire dont le paramètre de solubilité $\delta_a$ à 25°C est différent de 0 $(J/cm^3)^{½}$.

En particulier, par « *cire polaire* », on entend une cire dont la structure chimique est formée essentiellement, voire constituée, d'atomes de carbone et d'hydrogène, et comprenant au moins un hétéroatome fortement électronégatif tel qu'un atome d'oxygène, d'azote, de silicium ou de phosphore.

La définition et le calcul des paramètres de solubilité dans l'espace de solubilité tridimensionnel de HANSEN sont décrits dans l'article de C. M. HANSEN : « The three dimensionnal solubility parameters » J. Paint Technol. 39, 105 (1967).

Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ainsi que les forces d'interactions de KEESOM entre dipôles induits et dipôles permanents ;
- $\delta_h$ caractérise les forces d'interactions spécifiques (type liaisons hydrogène, acide/base, donneur/accepteur, etc.) ;
- $\delta_a$ est déterminé par l'équation : $\delta_a = (\delta_p^2 + \delta_h^2)^{½}$.

Les paramètres $\delta_p$, $\delta_h$, $\delta_D$ et $\delta_a$ sont exprimés en $(J/cm^3)^{½}$.

Les cires polaires peuvent notamment être hydrocarbonées, fluorées ou siliconées.

Préférentiellement, les cires polaires peuvent être hydrocarbonées ou fluorées.

Par « *cire siliconée* », on entend une huile comprenant au moins un atome de silicium, et notamment comprenant des groupes Si-O.

Par *« cire hydrocarbonée »*, on entend une cire formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Selon un premier mode de réalisation préféré, la cire polaire est une cire hydrocarbonée.

A titre de cire polaire hydrocarbonée, on préfère en particulier une cire choisie parmi les cires ester et les cires alcool.

Par « *cire ester* », on entend selon l'invention une cire comprenant au moins une fonction ester. Les cires esters peuvent en outre être hydroxylées.

Par « *cire alcool* », on entend selon l'invention une cire comprenant au moins une fonction alcool, c'est-à-dire comprenant au moins un groupe hydroxyle (OH) libre.

On peut notamment utiliser en tant que cire ester :

- les cires esters telles que celles choisies parmi :

  i) les cires de formule $R_1COOR_2$ dans laquelle $R_1$ et $R_2$ représentent des chaines aliphatiques linéaires, ramifiées ou cycliques dont le nombre d'atomes varie de 10 à 50, pouvant contenir un hétéroatome tel que O, N ou P et dont la température de point de fusion varie de 25 à 120°C. En particulier on peut utiliser comme cire ester un (hydroxystéaryloxy)stéarate d'alkyle en $C_{20}$-$C_{40}$ (le groupe alkyle comprenant de 20 à 40 atomes de carbone), seul ou en mélange ou un stéarate d'alkyle en $C_{20}$-$C_{40}$. De telles cires sont notamment vendues sous les dénominations « Kester Wax K 82 P® », « Hydroxypolyester K 82 P® », « Kester Wax K 80 P® », ou « KESTER WAX K82H » par la société KOSTER KEUNEN.

  On peut également utiliser un montanate (octacosanoate) de glycol et de butylène glycol tel que la cire LICOWAX KPS FLAKES (nom INCI : glycol montanate) commercialisée par la société Clariant.

  ii) le tétrastéarate de di-(triméthylol-1,1,1 propane), vendu sous la dénomination de Hest 2T-4S® par la société HETERENE.

  iii) les cires diesters d'un diacide carboxylique de formule générale $R^3$-(-OCO-$R^4$-COO-$R^5$), dans laquelle $R^3$ et $R^5$ sont identiques ou différents, de préférence identiques et représentent un groupe alkyle en $C_4$-$C_{30}$ (groupe alkyle comprenant de 4 à 30 atomes de carbone) et $R^4$ représente un groupe aliphatique en $C_4$-$C_{30}$ (groupe alkyle comprenant de 4 à 30 atomes de carbone) linéaire ou ramifié et pouvant contenir ou non 1 ou plusieurs insaturations. De préférence, le groupe aliphatique en $C_4$-$C_{30}$ est linéaire et insaturé.

  iv) On peut aussi citer les cires obtenues par hydrogénation catalytique d'huiles animales ou végétales ayant des chaînes grasses, linéaires ou ramifiées, en $C_8$-$C_{32}$, par exemple telles que l'huile de jojoba hydrogénée, l'huile de tournesol hydrogénée, l'huile de ricin hydrogénée, l'huile de coprah hydrogénée, ainsi que les cires obtenues par hydrogénation d'huile de ricin estérifiée avec l'alcool cétylique, telles que celles vendues sous les dénominations de Phytowax ricin 16L64® et 22L73® par la société SOPHIM. De telles cires sont décrites dans la demande FR-A-2792190. Comme cires obtenues par hydrogénation d'huile d'olive estérifiée avec l'alcool stéarylique, on peut citer celles vendues sous la dénomination « PHYTOWAX Olive 18 L 57 ».

  v) On peut également citer la cire d'abeille, la cire d'abeille synthétique, la cire d'abeille polyglycérolée, la cire de carnauba, la cire de candellila, la cire de lanoline oxypropylénée, la cire de son de riz, la cire d'Ouricury, la cire d'Alfa, la cire de fibres de liège, la cire de canne à sucre, la cire du Japon, la cire de sumac, la cire de montan, la cire d'Orange, la cire de Laurier, la cire de Jojoba hydrogénée.

Selon un mode de réalisation préféré, une composition conforme à l'invention comprend de la cire de candellila.

Selon un autre mode de réalisation, la cire polaire peut être une cire alcool.

A titre de cire alcool, on peut citer par exemple la cire Performacol 550-L Alcohol de New Phase Technologie, l'alcool stéarique et l'alcool cétylique.

Selon un second mode de réalisation, la cire polaire peut être siliconée comme la cire d'abeille siliconée.

La composition selon l'invention, si elle comprend une cire, qu'elle soit polaire ou apolaire, leur teneur ne dépasse pas 5 % en poids (soit comprise entre 0 et 5 % en poids), encore plus particulièrement, ne dépasse pas 2 % en poids (soit comprise entre 0 et 2 % en poids), par rapport au poids total de la composition.

## Huiles volatiles

La composition selon l'invention peut éventuellement comprendre au moins une huile volatile, en particulier hydrocarbonée ou siliconée.

Au sens de la présente invention, on entend par « *huile volatile* », une huile dont le point éclair est inférieur strictement à 49°C. Le point d'éclair est mesuré en coupelle fermée à l'aide d'un appareil dit de Pensky-Martens (*Closed Cup*).

Les huiles hydrocarbonées volatiles peuvent être choisies parmi les huiles hydrocarbonées ayant de 8 à moins de 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, et par exemple les huiles vendues sous les noms

commerciaux d'Isopars® ou de Permethyls®. Parmi les huiles volatiles, on peut également citer les parfums.

Comme huiles volatiles, on peut aussi utiliser les huiles de silicones volatiles, comme par exemple les huiles de silicones linéaires ou cycliques volatiles, notamment celles ayant une viscosité ≤ 8 centistokes (8 x $10^{-6}$ $m^2/s$), et ayant notamment de 2 à 10 atomes de silicium, et en particulier de 2 à 7 atomes de silicium, ces silicones comportant éventuellement des groupes alkyle ou alcoxy ayant de 1 à 10 atomes de carbone. Comme huile de silicone volatile utilisable dans l'invention, on peut citer notamment les diméthicones de viscosité inférieure ou égale à 6 cSt, l'heptaméthyl hexyltrisiloxane, l'heptaméthyloctyl trisiloxane, l'hexaméthyl disiloxane, l'octaméthyl trisiloxane, le décaméthyl tétrasiloxane, et leurs mélanges.

On peut également utiliser des huiles volatiles fluorées telles que le nonafluorométhoxybutane ou le perfluorométhylcyclopentane, et leurs mélanges.

De préférence, la composition selon l'invention, si elle comprend une ou plusieurs huiles volatiles, leur teneur ne dépasse pas 10 % en poids (soit comprise entre 0 et 10% en poids), de préférence ne dépasse pas 5 % en poids (soit comprise entre 0 et 5 % en poids), par rapport au poids total de la composition.

## Ingrédients cosmétiques usuels additionnels

Une composition selon l'invention peut comprendre en outre tout ingrédient cosmétique usuel pouvant être choisi notamment parmi les antioxydants, les parfums, les conservateurs, les neutralisants, les tensioactifs, les filtres solaires, les édulcorants, les vitamines, les hydratants, les émollients, les actifs hydrophiles ou lipophiles, les agents anti-radicaux libres, les séquestrants, et leurs mélanges.

La composition selon l'invention peut également comprendre au moins un polymère filmogène choisi parmi les polymères vinyliques comprenant au moins un motif dérivé de dendrimère carbosiloxane ; les copolymères éthyléniques séquencés filmogènes ; les alkylcelluloses ; les résines siliconées, les polyamides siliconés, ou leurs mélanges.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

Il est à noter qu'en fonction de son état liquide ou solide, l'ingrédient ajouté est introduit avec la phase pulvérulente ou avec la phase liante.

## Préparation de la composition

Les compositions selon l'invention peuvent être préparées selon le protocole qui suit.

Les matériaux formant la phase pulvérulente sont introduits directement dans un mélangeur malaxeur usuellement utilisé pour les produits pâteux et/ou poudreux, tels que les mélangeurs/granulateurs à turbine de type Baker-Perkins, les mélangeurs de type Pétrin ou les malaxeurs bi-vis continue type extrudeur-malaxeur BC21 ou BC45 de la société CLEXTRAL, suivant notamment les indications des fournisseurs.

L'organopolysiloxane élastomère réticulé véhiculé avec au moins une première huile est introduit avec la phase liante à la phase pulvérulente, ou séparément à la phase liante.

La phase liante comprend la ou les huiles non volatiles, au moins un composé pâteux, éventuellement au moins une cire et éventuellement au moins une huile volatile.

La phase liante et l'organopolysiloxane véhiculé dans au moins une première huile non volatile sont introduits sous agitation.

La température à laquelle la composition est préparée varie habituellement entre 20°C et 45°C.

La composition, qu'elle se trouve sous pulvérulente ou pâteuse, peut être pesée dans une coupelle ou récipient approprié, puis soumise à un pressage, par exemple sur Vetraco.

## Procédé de traitement mettant en oeuvre la composition

Les compositions selon l'invention peuvent être appliquées au doigt ou avantageusement à l'aide d'un applicateur mousse ou d'une éponge spécifique adapté(e) à la préhension à l'application d'une telle composition, sur les matières kératiniques, en particulier la peau et les lèvres, ou encore à l'aide d'un stylo-poussoir.

Dans les exemples qui suivent, les pourcentages en poids sont indiqués par rapport au poids total de la composition.

Les pourcentages en poids sont indiqués en matière première.

## Exemple 1

On prépare la composition à partir des ingrédients (quantités exprimées en pourcentage en poids de matière

première) :

| | | |
|---|---|---|
| D | Pentaérythrityl tétra-di-t-butyl hydroxyhydrocinnamate | 0,03 |
| A | Perlite | 4 |
| B | Blue 1 lake | 0,22 |
| B | Red 7 | 2,9 |
| B | Dioxyde de titane | 1,7 |
| B | Red 28 lake | 3,25 |
| D | Bis-diglyceryl polyacyladipate-2 (SOFTISAN 649 de Sasol) | 12,68 |
| D | Diisostéaryl malate | 5,76 |
| D | Isostéaryl isostéarate | 2,45 |
| B | Mica / lauroyl lysine | 2,67 |
| A | Mica | qs |
| D | Polyisobutène hydrogéné (PARLEAM de NOF) | 5,38 |
| A | HDI/trimethylol hexyllactone crosspolymer (PLASTIC POWDER D400 de Toshiki Pigment) | 3,67 |
| D | Phényl triméthicone (DOW CORNING® 556 COSMETIC GRADE FLUID de Dow Corning) | 3,69 |
| D | Dimethicone et dimethicone crosspolymer (DOW CORNING 9041 SILICONE ELASTOMER BLEND, de Dow Corning) | 29,5 |
| D | Caprylyl glycol | 0,5 |

On introduit les phases A et B dans un mélangeur de type Baker et on mélange pendant 3 minutes 30 (vitesse de pale 3000 trs/min / vitesse emoteur 2700 trs/min).

On obtient un mélange C que l'on transfère dans un pétrin.

On prépare un mélange homogène des ingrédients de la phase D à 25°C et on l'ajoute petit à petit au mélange C, sous faible agitation, jusqu'à homogénéisation complète de la composition.

On obtient une pâte homogène et cohésive, que l'on conditionne en coupelle. Elle est souple à la prise et à l'application.

Le dépôt est fin, mat et confortable. Il présente de plus une bonne tenue.

**Exemples 2**

On prépare les compositions dont les ingrédients sont reportés dans le tableau ci-dessous (quantités exprimées en pourcentages en poids de matière première sauf indication contraire) :

| Phase | Ingrédients | Composition 1 invention | Composition 2 comparative |
|---|---|---|---|
| D | Pentaérythrityl tétra-di-t-butyl hydroxyhydrocinnamate (TINOGARD TT, BASF) | 0,02 | 0,02 |
| A | Talc | 3,34 | 3,342 |
| B | Dioxide de titane | 4,55 | 4,55 |
| B | Yellow 6 lake | 4 | 4 |
| B | Red 28 lake | 2,5 | 2,5 |
| B | Iron oxides | 0,14 | 0,14 |
| D | Bis-diglycéryl polyacyladipate-2 (SOFTISAN 649, Cremer Oleo) | 10,53 | 8,41 |
| D | Diisostéaryl malate | 4,79 | 3,82 |

(suite)

| Phase | Ingrédients | Composition 1 invention | Composition 2 comparative |
|---|---|---|---|
| D | Isostéaryl isostéarate | 2,03 | 1,62 |
| B | Mica /Lauroyl lysine | 2,67 | 2,67 |
| B | Mica | 19,14 | 19,14 |
| D | Parfum | 0,1 | 0,1 |
| D | Polyisobutène hydrogéné (PARLEAM de NOF) | 4,47 | 3,57 |
| D. | Polyéthylène (ASENSA SC 211, Honeywell) | 1,1 | 3,85 |
| D | Polyéthylène (PERFORMALENE 500-L POLYETHYLENE, New Phase technologies) | 0,9 | 3,15 |
| B | HDI/trimethylol hexyllactone crosspolymer (PLASTIC POWDER D400 de Toshiki Pigment) | 3,67 | 3,67 |
| D | Phenyl trimethicone (DOW CORNING® 556 COSMETIC GRADE FLUID de Dow Corning) | 3,06 | 2,45 |
| B | Vinyl dimethicone/methicone silsesquioxane crosspolymer (KSP100 de Shin Etsu) | 2 | 2 |
| D | Dimethicone (and) Dimethicone crosspolymer (DOW CORNING 9041 SILICONE ELASTOMER BLEND; Dow Corning) | 29 | 29 |
| D | Ethylhexylglycérine | 0,5 | 0,5 |
| D | Pentylène glycol | 1 | 1 |
| D | Caprylyl glycol | 0,5 | 0,5 |

On introduit les phases A et B dans un mélangeur de type Baker et on mélange pendant 3 minutes 30 (vitesse de pale 3000 trs/min / vitesse emoteur 2700 trs/min).

On obtient un mélange C que l'on transfère dans un pétrin.

On prépare un mélange homogène des ingrédients de la phase D en chauffant jusqu'à homogénéisation complète de la phase D et on l'ajoute petit à petit au mélange C, sous faible agitation, jusqu'à homogénéisation complète de la composition.

La composition est ensuite conditionnée en coupelle.

Dans le cas de la composition 1 selon l'invention, comprenant de 2% en poids de cire, on obtient une pâte homogène, stable, sans exsudation d'huile, souple et cohésive. La prise est facile, l'application facile et agréable.

On obtient un dépôt fin, homogène couvrant et mat, très confortable. Il présente également une bonne tenue de la couleur et de la matité.

Dans le cas de la composition 2 comparative comprenant une teneur en cire de 7 % en poids, la composition obtenue n'est plus sous forme de pâte, elle est très dure et très difficile à prélever.

## Revendications

**1.** Procédé de préparation d'une composition anhydre sous forme de poudre ou sous forme de pâte, dans lequel on met en oeuvre les étapes suivantes :

- On prépare 30 à 65 % en poids, par rapport au poids de la composition, de particules solides organiques, minérales ou composites, ainsi que leurs mélanges ;
- On prépare 10 à 40 % en poids par rapport au poids de la composition, d'un mélange comprenant au moins un élastomère d'organopolysiloxane véhiculé dans au moins une première huile non volatile, siliconée ou hydrocarbonée,
- On prépare 10 à 45 % en poids, par rapport au poids de la composition, d'une phase liante organique comprenant

au moins une deuxième huile hydrocarbonée ou siliconée, non volatile différente ou non de la première huile, au moins un composé pâteux à une teneur comprise entre 5 et 25 % en poids, par rapport au poids de la composition, éventuellement au moins une cire et éventuellement au moins une huile volatile ; la teneur en cire ne dépassant pas 5 % en poids, par rapport au poids de la composition,

- On introduit sous agitation, la phase liante et ledit élastomère d'organopolysiloxane véhiculé, aux particules solides ; l'élastomère d'organopolysiloxane véhiculé étant introduit avec la phase liante ou séparément de celle-ci,

étant entendu que l'on comprend par "cire" un composé lipophile, solide à 25°C, à changement d'état solide/liquide réversible, ayant un point de fusion supérieur ou égal à 30°C pouvant aller jusqu'à 120°C,
étant entendu que l'on comprend par "composé pâteux" un composé à changement d'état solide/liquide réversible, présentant à l'état solide une organisation cristalline anisotrope, et comportant à la température de 23°C une fraction liquide et une fraction solide,
et étant entendu que l'on comprend par « non volatile » un composé dont le point éclair est supérieur ou égal à 49°C.

**2.** Procédé selon la revendication précédente, **caractérisé en ce que** l'élastomère d'organopolysiloxane véhiculé dans au moins une première huile, est non émulsionnant.

**3.** Procédé selon l'une quelconque des revendications précédentes, **caractérisé en ce que** l'élastomère d'organopolysiloxane véhiculé dans au moins une première huile, est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence de catalyseur platine (C).

**4.** Procédé selon l'une quelconque des revendications précédentes, **caractérisé en ce que** la teneur en mélange d'élastomère(s) d'organopolysiloxane(s) véhiculé(s) dans au moins une première huile non volatile, représente de 15 à 35 % en poids, par rapport au poids de la composition.

**5.** Procédé selon l'une quelconque des revendications précédentes, **caractérisé en ce que** teneur en élastomère d'organopolysiloxane dans ledit mélange est telle que la teneur en élastomère d'organopolysiloxane dans la composition, exprimée en élastomère d'organopolysiloxane, varie de 2 à 10 % en poids de la composition.

**6.** Procédé selon l'une quelconque des revendications précédentes, **caractérisé en ce que** la ou les particules solides sont choisies parmi les particules solides colorées telles que les pigments organiques, minéraux ou composites, les nacres, ou parmi les charges, organiques, minérales ou mixtes, ainsi que leurs mélanges.

**7.** Procédé selon la revendication précédente, **caractérisé en ce que** la teneur en particule(s) colorée(s) représente de 5 à 25 % en poids, par rapport au poids de la composition.

**8.** Procédé selon la revendication 7, **caractérisé en ce que** la ou les charges organiques, minérales ou mixtes, sont choisies parmi le talc ; le mica ; la silice, pyrogénée ou non, éventuellement traitée hydrophile ou hydrophobe ; la perlite ; le kaolin ; la bentone ; l'amidon ; le nitrure de bore ; les microsphères creuses polymériques ; les microbilles de résine de silicone ; le carbonate de calcium précipité ; le carbonate et l'hydrocarbonate de magnésium ; l'hydroxyapatite ; les microsphères de silice creuses ; les particules de polyorganosiloxanes élastomères; les particules de polyuréthanne ; les poudres de polyamide, de polyéthylène, de polyméthacrylate de méthyle, les poudres de polytétrafluoroéthylène, de copolymères d'acide acrylique, la lauroyl lysine, les microsphères creuses polymériques, les microbilles de résine de silicone, les cires micronisées synthétiques ou naturelles, les savons métalliques dérivés d'acides organiques carboxyliques ayant de 8 à 22 atomes de carbone ; les microcapsules de verre ou de céramique ; ou leurs mélanges.

**9.** Procédé selon l'une quelconque des revendications 7 ou 8, **caractérisé en ce que** la teneur en charge(s) organique(s), minérale(s) ou composite(s), ou leurs mélanges, représente de 10 à 40 % en poids, par rapport au poids de la composition.

**10.** Procédé selon l'une quelconque des revendications précédentes, **caractérisé en ce qu'**au moins une première huile et/ou au moins une deuxième huile est choisie parmi les huiles non volatiles hydrocarbonées non volatiles choisies parmi les huiles hydrocarbonées non volatiles polaires, apolaires ou leurs mélanges.

**11.** Procédé selon la revendication précédente, **caractérisé en ce que** la ou les huiles hydrocarbonées non volatiles apolaires sont choisies parmi l'huile de paraffine ou ses dérivés; le squalane ; l'isohexadécane ; l'isoeicosane ; l'huile de naphtalène ; les polybutènes, hydrogénés ou non ; les polyisobutènes hydrogénés ou non ; les copolymères décène/butène, les copolymères polybutène/polyisobuténe ; les polydécènes, hydrogénés ou non ; et leurs mélanges ; et de préférence parmi les polybutènes, hydrogénés ou non, les polyisobutènes hydrogénés ou non, les polydécènes hydrogénés ou non, ainsi que leurs mélanges.

**12.** Procédé selon la revendication 10, **caractérisé en ce que** la ou les huiles hydrocarbonées non volatiles polaires sont choisies parmi les alcools en $C_{10}$-$C_{26}$; les huiles esters; les huiles hydrocarbonées végétales; les copolymères vinylpyrrolidone/1-héxadécène ; les acides gras en $C_{12}$-$C_{26}$ ; les carbonates de di-alkyle ; leurs mélanges, et de préférence parmi les monoesters, les diesters, hydroxylés ou non, comprenant au moins 18 atomes de carbone au total, ainsi que leurs mélanges.

**13.** Procédé selon l'une quelconque des revendications précédentes, **caractérisé en ce qu'**au moins une première huile et/ou au moins une deuxième huile est choisie parmi les huiles non volatiles sificonées non phénylées, parmi les huiles siliconées phénylées possédant ou non un fragment diméthicone, ou leurs mélanges.

**14.** Procédé selon l'une quelconque des revendications précédentes, **caractérisé en ce que** la phase liante comprend, en tant que deuxièmes huiles, différentes de la première huile, au moins une huile hydrocarbonée non volatile, et au moins une huile siliconée non volatile.

**15.** Procédé selon l'une quelconque des revendications précédentes, **caractérisé en ce que** la teneur en composé(s) hydrocarboné(s) non volatile(s), en composé(s) siliconé(s) non volatile(s), ou leurs mélanges, varie de 5 à 40 % en poids, de préférence de 10 à 20 % en poids par rapport au poids de la composition.

**16.** Procédé selon l'une quelconque des revendications précédentes, **caractérisé en ce que** la ou les premières huiles sont choisies parmi les huiles non volatiles siliconées non phénylées, parmi les huiles siliconées phénylées possédant ou non un fragment diméthicone, ou leurs mélanges.

**17.** Procédé selon l'une quelconque des revendications précédentes, **caractérisé en ce que** la phase liante comprend au moins au moins un composé hydrocarboné ou siliconé pâteux à 25°C et pression atmosphérique choisi parmi :

- la lanoline et ses dérivés,
- les composés siliconés polymères ou non polymères,
- les composés fluorés polymères ou non polymères,
- les polymères vinyliques, notamment les homopolymères d'oléfines, les copolymères d'oléfines, les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters,
- et leurs mélanges,

et de préférence sont choisis parmi la lanoline et ses dérivés, les esters d'oligomères de glycérol, les beurres d'origine végétale, les huiles végétales totalement ou partiellement hydrogénées, les esters d'huiles de ricin hydrogéné, ou leurs mélanges.

**18.** Procédé selon l'une quelconque des revendications précédentes, **caractérisé en ce que** la teneur en composé(s) pâteux à 25°C et pression atmosphérique représente 5 à 15 % en poids, par rapport à la composition.

**19.** Procédé selon l'une quelconque des revendications précédentes, **caractérisé en ce que** la composition comprend au moins une huile, hydrocarbonée ou siliconée, volatile, en particulier à une teneur ne dépassant pas 10 % en poids, de préférence ne dépassant pas 5 % en poids, par rapport au poids de la composition.

**20.** Procédé de maquillage et/ou de soin des lèvres dans lequel on applique la composition obtenue selon l'une quelconque des revendications précédentes.

## Patentansprüche

1. Verfahren zur Herstellung einer wasserfreien Zusammensetzung in der Form eines Pulvers oder in der Form einer Paste, wobei die folgenden Schritte durchgeführt werden:

   - 30 bis 65 Gew.-%, bezogen auf das Gewicht der Zusammensetzung, organischer, mineralischer oder zusammengesetzter fester Partikel sowie ihrer Mischungen werden hergestellt;
   - 10 bis 40 Gew.-%, bezogen auf das Gewicht der Zusammensetzung, einer Mischung werden hergestellt, umfassend mindestens ein Organopolysiloxan-Elastomer in einem Träger mindestens eines ersten nicht-flüchtigen Silikon-oder Kohlenwasserstofföls;
   - 10 bis 45 Gew.-%, bezogen auf das Gewicht der Zusammensetzung, einer organischen Bindephase werden hergestellt, umfassend mindestens ein zweites nicht-flüchtiges Kohlenwasserstoff- oder Silikonöl, das von dem ersten Öl verschieden ist oder nicht, mindestens eine pastenförmige Verbindung mit einem Gehalt zwischen 5 und 25 Gew.-%, bezogen auf das Gewicht der Zusammensetzung, gegebenenfalls mindestens ein Wachs und gegebenenfalls mindestens ein flüchtiges Öl; wobei der Gehalt an Wachs 5 Gew.-%, bezogen auf das Gewicht der Zusammensetzung, nicht überschreitet;
   - unter Rühren werden die Bindephase und das Organopolysiloxan-Elastomer in dem Träger den festen Partikeln zugesetzt; wobei das Organopolysiloxan-Elastomer in dem Träger mit der Bindephase oder getrennt von dieser eingebracht wird;

   mit der Maßgabe, dass unter "Wachs" eine lipophile Verbindung, die bei 25°C fest ist, mit einer reversiblen Änderung des festen/flüssigen Zustands mit einem Schmelzpunkt von größer oder gleich 30°C, der bis 120°C gehen kann, verstanden wird; mit der Maßgabe, dass unter "pastenförmige Verbindung" eine Verbindung mit einer reversiblen Änderung des festen/flüssigen Zustands verstanden wird, die im festen Zustand eine anisotrope kristalline Organisation aufweist und bei der Temperatur von 23°C eine flüssige Fraktion und eine feste Fraktion aufweist; und
   mit der Maßgabe, dass unter "nicht-flüchtig" eine Verbindung verstanden wird, deren Flammpunkt größer oder gleich 49°C ist.

2. Verfahren nach dem vorhergehenden Anspruch,
   **dadurch gekennzeichnet, dass** das Organopolysiloxan-Elastomer in mindestens einem ersten Trägeröl nicht emulgierend ist.

3. Verfahren nach einem der vorhergehenden Ansprüche,
   **dadurch gekennzeichnet, dass** das Organopolysiloxan-Elastomer in mindestens einem ersten Trägeröl durch eine Vernetzungsadditionsreaktion von (A) Diorganopolysiloxan, enthaltend mindestens zwei Wasserstoffe, die jeweils an ein Silicium gebunden sind, und (B) Diorganopolysiloxan mit mindestens zwei ethylenisch ungesättigten Gruppen, die an Silicium gebunden sind, insbesondere in Anwesenheit eines Platin-Katalysators (C) erhalten wird.

4. Verfahren nach einem der vorhergehenden Ansprüche,
   **dadurch gekennzeichnet, dass** der Gehalt der Mischung von Organopolysiloxan-Elastomer(en) in dem mindestens einen nicht-flüchtigen Trägeröl von 15 bis 35 Gew.-%, bezogen auf das Gewicht der Zusammensetzung, beträgt.

5. Verfahren nach einem der vorhergehenden Ansprüche,
   **dadurch gekennzeichnet, dass** der Gehalt von Organopolysiloxan-Elastomer in der Mischung derart ist, dass der Gehalt an Organopolysiloxan-Elastomer in der Mischung, ausgedrückt in Organopolysiloxan-Elastomer, von 2 bis 10 Gew.-% der Zusammensetzung variiert.

6. Verfahren nach einem der vorhergehenden Ansprüche,
   **dadurch gekennzeichnet, dass** das feste oder die festen Partikel ausgewählt werden aus gefärbten festen Partikeln, wie organischen Pigmenten, Mineralien oder Verbundstoffen, Perlmutt, oder aus organischen, mineralischen oder gemischten Chargen, sowie ihren Mischungen.

7. Verfahren nach dem vorhergehenden Anspruch,
   **dadurch gekennzeichnet, dass** der Gehalt an gefärbtem(n) Partikel(n) von 5 bis 25 Gew.-%, bezogen auf das Gewicht der Zusammensetzung, beträgt.

8. Verfahren nach Anspruch 7,
   **dadurch gekennzeichnet, dass** die organische(n), mineralische(n) oder gemischte(n) Charge(n) ausgewählt wird

(werden) aus: Talk; Mica, Siliciumdioxid, pyrogenisiert oder nicht, gegebenenfalls hydrophil oder hydrophob behandelt; Perlit; Kaolin; Benton; Stärke; Bornitrid; polymeren hohlen Mikrosphären; Mikrokügelchen aus Silikonharz; gefälltem Calciumcarbonat; Magnesiumcarbonat und -hydrocarbonat; Hydroxyapatit; hohlen Mikrosphären aus Siliciumdioxid; Partikeln von Polyorganosiloxan-Elastomeren; Partikeln von Polyurethan; Pulvern von Polyamid, Polyethylen, Polymethylmetacrylat, Pulvern von Polytetrafluorethylen, Copolymeren von Acrylsäure, Lauroyllysin, hohlen polymeren Mikrosphären, Mikrokügelchen aus Silikonharz, mikronisierten synthetischen oder natürlichen Wachsen, Metallseifen, die von organischen Carbonsäuren mit 8 bis 22 Kohlenstoffatomen stammen; Mikrokapseln von Glas oder Keramik; oder ihren Mischungen.

**9.** Verfahren nach einem der Ansprüche 7 oder 8,
**dadurch gekennzeichnet, dass** der Gehalt an mineralischer(n) organischer(n) oder zusammengesetzter(n) Charge(n) oder ihren Mischungen 10 bis 40 Gew.-%, bezogen auf das Gewicht der Zusammensetzung, beträgt.

**10.** Verfahren nach einem der vorhergehenden Ansprüche,
**dadurch gekennzeichnet, dass** mindestens ein erstes Öl und/oder mindestens ein zweites Öl unter den nichtflüchtigen Ölen von nicht-flüchtigem Kohlenwasserstoff aus polaren, apolaren nicht-flüchtigen Kohlenwasserstoffölen oder ihren Mischungen ausgewählt wird/werden.

**11.** Verfahren nach dem vorhergehenden Anspruch,
**dadurch gekennzeichnet, dass** das oder die nicht-flüchtigen apolaren Kohlenwasserstofföle ausgewählt werden aus Paraffinöl oder seinen Derivaten; Squalan; Isohexadecan; Isoeicosan; Naphthalinöl; Polybutenen, hydrogeniert oder nicht; Polyisobutenen, hydrogeniert oder nicht; Copolymeren von Decen/Buten, Copolymeren von Polybuten/Polyisobuten; Polydecenen, hydrogeniert oder nicht; und ihren Mischungen; und vorzugsweise aus Polybutenen, hydrogeniert oder nicht, Polyisobutenen, hydrogeniert oder nicht, Polydecenen, hydrogeniert oder nicht, sowie ihren Mischungen.

**12.** Verfahren nach Anspruch 10,
**dadurch gekennzeichnet, dass** das oder die nicht-flüchtigen polaren Kohlenwasserstofföle ausgewählt werden aus $C_{10}$-$C_{26}$-Alkoholen; Esterölen; pflanzlichen Kohlenwasserstoffölen; Copolymeren von Vinylpyrrolidon/1-Hexadecen; $C_{12}$-$C_{26}$-Fettsäuren; Dialkylcarbonaten; ihren Mischungen, und vorzugsweise aus Monoestern, Diestern, hydroxyliert oder nicht, mit insgesamt mindestens 18 Kohlenstoffatomen, sowie ihren Mischungen.

**13.** Verfahren nach einem der vorhergehenden Ansprüche,
**dadurch gekennzeichnet, dass** mindestens ein erstes Öl und/oder mindestens ein zweites Öl ausgewählt wird aus nicht-flüchtigen, nicht-phenylierten Silikonölen, aus phenylierten Silikonölen mit einem Dimethicon-Fragment oder nicht, oder ihren Mischungen.

**14.** Verfahren nach einem der vorhergehenden Ansprüche,
**dadurch gekennzeichnet, dass** die Bindephase, als zweite Öle, die von dem ersten Öl verschieden sind, mindestens ein nicht-flüchtiges Kohlenwasserstofföl und mindestens ein nicht-flüchtiges Silikonöl umfasst.

**15.** Verfahren nach einem der vorhergehenden Ansprüche,
**dadurch gekennzeichnet, dass** der Gehalt an nichtflüchtiger(n) Kohlenwasserstoffverbindung(en), an nicht-flüchtiger(n) Silikonverbindung(en) oder ihren Mischungen von 5 bis 40 Gew.-%, vorzugsweise von 10 bis 20 Gew.-%, bezogen auf das Gewicht der Zusammensetzung, variiert.

**16.** Verfahren nach einem der vorhergehenden Ansprüche,
**dadurch gekennzeichnet, dass** das oder die ersten Öle ausgewählt werden aus nicht-flüchtigen nichtphenylierten Silikonölen, aus phenylierten Silikonölen mit einem Dimethicon-Fragment oder nicht, oder ihren Mischungen.

**17.** Verfahren nach einem der vorhergehenden Ansprüche,
**dadurch gekennzeichnet, dass** die Bindephase mindestens eine Kohlenwasserstoff- oder Silikonverbindung umfasst, die bei 25°C und Atmosphärendruck pastenförmig ist, ausgewählt aus:

- Lanolin und seinen Derivaten,
- polymeren oder nicht-polymeren Silikonverbindungen,
- polymeren oder nicht-polymeren Fluorverbindungen,
- Vinylpolymeren, insbesondere Olefinhomopolymeren, Olefin-Copolymeren, Homopolymeren und Copolymeren von hydrogenierten Dienen,
- linearen oder verzweigten Oligomeren, Homo-oder Copolymeren von Alkyl(meth)acrylaten vorzugsweise mit einer $C_8$-$C_{30}$-Alkylgruppe,
- Homooligomeren und Copolymeren von Vinylestern mit $C_8$-$C_{30}$-Alkylgruppen,
- Homooligomeren und Copolymeren von Vinylethern mit $C_8$-$C_{30}$-Alkylgruppen,
- lipolöslichen Polyethern, die aus der Polyveretherung zwischen einem oder mehreren $C_2$-$C_{100}$-, vorzugsweise $C_2$-$C_{50}$-Diolen erhalten werden,
- Estern und Polyestern,
- und ihren Mischungen,

und vorzugsweise ausgewählt aus Lanolin und seinen Derivaten, Oligomerestern von Glycerin, Butter pflanzlichen Ursprungs, vollständig oder teilweise hydrogenierten Pflanzenölen, Estern von hydrogenierten Rizinusölen, oder ihren Mischungen.

**18.** Verfahren nach einem der vorhergehenden Ansprüche,
**dadurch gekennzeichnet, dass** der Gehalt an bei 25°C und Atmosphärendruck pastenförmiger(n) Verbindung(en) 5 bis 15 Gew.-%, bezogen auf die Zusammensetzung, beträgt.

**19.** Verfahren nach einem der vorhergehenden Ansprüche,
**dadurch gekennzeichnet, dass** die Zusammensetzung mindestens ein flüchtiges Kohlenwasserstoff- oder Silikonöl umfasst, insbesondere mit einem Gehalt, der 10 Gew.-% nicht überschreitet, vorzugsweise 5 Gew.-% nicht überschreitet, bezogen auf das Gewicht der Zusammensetzung.

**20.** Verfahren zum Schminken oder zur Lippenpflege, wobei die Zusammensetzung, die nach einem der vorhergehenden Ansprüche erhalten wird, angewendet wird.

## Claims

**1.** Process for preparing an anhydrous composition in powder form or in paste form, in which the following steps are performed:

- from 30% to 65% by weight, relative to the weight of the composition, of organic, mineral or composite solid particles, and also mixtures thereof, are prepared,
- 10% to 40% by weight, relative to the weight of the composition, of a mixture comprising at least one organopolysiloxane elastomer conveyed in at least a first silicone or hydrocarbon-based non-volatile oil is prepared,
- 10% to 45% by weight, relative to the weight of the composition, of an organic binder phase comprising at least a second hydrocarbon-based or silicone non-volatile oil, identical to or different from the first oil, is prepared, at least one pasty compound having a content of between 5% and 25% by weight, relative to the weight of the composition, optionally at least one wax and optionally at least one volatile oil; the wax content not exceeding 5% by weight, relative to the weight of the composition,
- the binder phase and said conveyed organopolysiloxane elastomer are introduced into the solid particles with stirring; the conveyed organopolysiloxane elastomer being introduced with the binder phase or separately therefrom, it being understood that the term "wax" means a lipophilic compound, which is solid at 25°C, with a reversible solid/liquid change of state, which has a melting point of greater than or equal to 30°C that may be up to 120°C, it being understood that the term "*pasty* compound" means a compound that undergoes a reversible solid/liquid change of state, having anisotropic crystal organization in the solid state, and comprising, at a temperature of 23°C, a liquid fraction and a solid fraction, and it being understood that the term "non-volatile" means a compound whose flash point is greater than or equal to 49°C.

**2.** Process according to the preceding claim, **characterized in that** the organopolysiloxane elastomer conveyed in at least a first oil is non-emulsifying.

**3.** Process according to either of the preceding claims, **characterized in that** the organopolysiloxane elastomer conveyed in at least a first oil is obtained by crosslinking addition reaction (A) of diorganopolysiloxane containing at least two hydrogens each bonded to a silicon, and (B) of diorganopolysiloxane containing at least two ethylenically unsaturated groups bonded to silicon, especially in the presence of a platinum catalyst (C).

4. Process according to any one of the preceding claims, **characterized in that** the content of mixture of organopolysiloxane elastomer (s) conveyed in at least a first non-volatile oil represents from 15% to 35% by weight relative to the weight of the composition.

5. Process according to any one of the preceding claims, **characterized in that** the organopolysiloxane elastomer content in said mixture is such that the organopolysiloxane elastomer content in the composition, expressed as organopolysiloxane elastomer, ranges from 2% to 10% by weight of the composition.

6. Process according to any one of the preceding claims, **characterized in that** the solid particle(s) are chosen from coloured solid particles such as organic, mineral or composite pigments, nacres, or from organic, mineral or mixed fillers, and also mixtures thereof.

7. Process according to the preceding claim, **characterized in that** the content of coloured particle(s) represents from 5% to 25% by weight, relative to the weight of the composition.

8. Process according to Claim 7, **characterized in that** the organic, mineral or mixed filler(s) are chosen from talc; mica; fumed or non-fumed silica, which has optionally undergone a hydrophilic or hydrophobic treatment; perlite; kaolin; bentonite; starch; boron nitride; hollow polymer microspheres; silicone resin microbeads; precipitated calcium carbonate; magnesium carbonate, magnesium hydrocarbonate; hydroxyapatite; hollow silica microspheres; polyorganosiloxane elastomer particles; polyurethane particles; polyamide, polyethylene or polymethyl methacrylate powders, polytetrafluoroethylene powders, acrylic acid copolymer powders, lauroyllysine, hollow polymer microspheres, silicone resin microbeads, synthetic or natural micronized waxes, metal soaps derived from organic carboxylic acids containing from 8 to 22 carbon atoms; glass or ceramic microcapsules; or mixtures thereof.

9. Process according to either of Claims 7 and 8, **characterized in that** the content of organic, mineral or composite filler(s), or mixtures thereof, represents from 10% to 40% by weight, relative to the weight of the composition.

10. Process according to any one of the preceding claims, **characterized in that** at least a first oil and/or at least a second oil is chosen from non-volatile hydrocarbon-based oils that are non-volatile chosen from polar or apolar non-volatile hydrocarbon-based oils, or mixtures thereof.

11. Process according to the preceding claim, **characterized in that** the apolar non-volatile hydrocarbon-based oil(s) are chosen from liquid paraffin or derivatives thereof, squalane, isohexadecane, isoeicosane, naphthalene oil, hydrogenated or non-hydrogenated polybutenes, hydrogenated or non-hydrogenated polyisobutenes, decene/butene copolymers, polybutene/polyisobutene copolymers, hydrogenated or non-hydrogenated polydecenes, and mixtures thereof, and preferably from hydrogenated or non-hydrogenated polybutenes, hydrogenated or non-hydrogenated polyisobutenes and hydrogenated or non-hydrogenated polydecenes, and also mixtures thereof.

12. Process according to Claim 10, **characterized in that** the polar non-volatile hydrocarbon-based oil(s) are chosen from $C_{10}$-$C_{26}$ alcohols; ester oils; hydrocarbon-based plant oils; vinylpyrrolidone/1-hexadecene copolymers; $C_{12}$-$C_{26}$ fatty acids; dialkyl carbonates; mixtures thereof, and preferably from hydroxylated or non-hydroxylated monoesters or diesters, comprising at least 18 carbon atoms in total, and also mixtures thereof.

13. Process according to any one of the preceding claims, **characterized in that** at least a first oil and/or at least a second oil is chosen from non-phenyl non-volatile silicone oils, from phenyl silicone oils optionally bearing a dimethicone fragment, or mixtures thereof.

14. Process according to any one of the preceding claims, **characterized in that** the binder phase comprises, as second oils, different from the first oil, at least one non-volatile hydrocarbon-based oil, and at least one non-volatile silicone oil.

15. Process according to any one of the preceding claims, **characterized in that** the content of non-volatile hydrocarbon-based compound(s), of non-volatile silicone compound(s), or mixtures thereof, ranges from 5% to 40% by weight and preferably from 10% to 20% by weight relative to the weight of the composition.

16. Process according to any one of the preceding claims, **characterized in that** the first oil(s) are chosen from non-phenyl non-volatile silicone oils, from phenyl silicone oils optionally bearing a dimethicone fragment, or mixtures thereof.

**17.** Process according to any one of the preceding claims, **characterized in that** the binder phase comprises at least one hydrocarbon-based or silicone compound that is pasty at 25°C and atmospheric pressure, chosen from:

- lanolin and derivatives thereof,
- polymeric or non-polymeric silicone compounds,
- polymeric or non-polymeric fluoro compounds,
- vinyl polymers, especially olefin homopolymers, olefin copolymers, or hydrogenated diene homopolymers and copolymers,
- linear or branched oligomers, homopolymers or copolymers of alkyl (meth)acrylates preferably containing a $C_8$-$C_{30}$ alkyl group,
- oligomers, homopolymers and copolymers of vinyl esters containing $C_8$-$C_{30}$ alkyl groups,
- oligomers, homopolymers and copolymers of vinyl ethers containing $C_8$-$C_{30}$ alkyl groups,
- liposoluble polyethers resulting from the polyetherification between one or more $C_2$-$C_{100}$ and preferably $C_2$-$C_{50}$ diols,
- esters and polyesters,
- and mixtures thereof,

and are preferably chosen from lanolin and derivatives thereof, esters of glycerol oligomers, butters of plant origin, totally or partially hydrogenated plant oils, esters of hydrogenated castor oils, or mixtures thereof.

**18.** Process according to any one of the preceding claims, **characterized in that** the content of compound(s) that are pasty at 25°C and atmospheric pressure represents from 5% to 15% by weight, relative to the composition.

**19.** Process according to any one of the preceding claims, **characterized in that** the composition comprises at least one hydrocarbon-based or silicone volatile oil, in particular in a content not exceeding 10% by weight and preferably not exceeding 5% by weight, relative to the weight of the composition.

**20.** Process for making up and/or caring for the lips, in which the composition obtained according to any one of the preceding claims is applied.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 12066457 A
- US 20130164235 A
- US 5236986 A
- US 5412004 A
- US 5837793 A
- US 5811487 A
- EP 1086683 A
- US 5538793 A
- FR 0853634
- FR 2792190 A

### Littérature non-brevet citée dans la description

- International Cosmetic Ingrédient Dictionnary and Handbook. The Cosmetic, Toiletry, and Fragrance Association, 1997, 371-386, 524-528
- Des silices ainsi traitées sont dénommées. Silica diméthyl silylate. CTFA, 1995
- **C. M. HANSEN.** The three dimensionnal solubility parameters. *J. Paint Technol.,* 1967, vol. 39, 105